# EUROPEAN PATENT SPECIFICATION

(11) **EP 2 830 596 B1**
(45) Date of publication and mention of the grant of the patent: **30.12.2020**
(21) Application number: 13769005.3
(22) Date of filing: 29.03.2013
(51) Int. Cl.: A61K 9/127, G01N 33/92, C12N 15/88, C12N 15/11

(54) **LIPID-DERIVED NEUTRAL NANOPARTICLES**
AUS LIPIDEN GEWONNENE NEUTRALE NANOPARTIKEL
NANOPARTICULES NEUTRES DÉRIVÉES DE LIPIDES

(30) Priority: 29.03.2012 US 201261617478 P
(43) Date of publication of application: 04.02.2015
(73) Proprietor: Translate Bio, Inc., Lexington, MA 02421 (US)
(72) Inventor: DEROSA, Frank, Chelmsford, MA 01824 (US); GUILD, Braydon, Charles, Concord, MA 01742 (US); HEARTLEIN, Michael, Boxborough, MA 01719 (US)
(74) Representative: Carpmaels & Ransford LLP
(86) International application number: PCT/US2013/034604
(87) International publication number: WO 2013/149141

(56) References cited:
- WO-A1-01/05375
- WO-A1-2012/170889
- US-A- 5 965 434
- US-A- 5 965 434
- US-A1- 2003 082 154
- US-A1- 2009 270 481
- US-A1- 2011 092 739
- TANG ET AL: "Introduction of a Disulfide Bond into a Cationic Lipid Enhances Transgene Expression of Plasmid DNA", BIOCHEMICAL AND BIOPHYSICAL RESEARCH COMMUNICATIONS, ACADEMIC PRESS INC. ORLANDO, FL, US, vol. 242, no. 1, 6 January 1998 (1998-01-06), pages 141-145, XP005100898, ISSN: 0006-291X, DOI: 10.1006/BBRC.1997.7923
- WETZER B ET AL: "Reducible cationic lipids for gene transfer", BIOCHEMICAL JOURNAL, PORTLAND PRESS LTD, GB, vol. 356, no. Pt 3, 15 June 2001 (2001-06-15), pages 747-756, XP002407694, ISSN: 0264-6021, DOI: 10.1042/0264-6021:3560747
- WETZER ET AL.: 'Reducible cationic lipids for gene transfer' BIOCHEM J vol. 356, 2001, pages 745 - 756, XP002407694

## Description

### BACKGROUND

The use of liposomal delivery vehicles to facilitate the site-specific delivery of therapeutic agents represents a rapidly emerging field of drug delivery; however, the efficient delivery of therapeutic agents to targeted cells and tissues, as well as the subsequent transfection of such targeted cells and tissues remains a technical challenge. Despite the availability of multiple lipids and liposomal-based delivery systems to facilitate the delivery of therapeutic agents to target cells and tissues, many challenges still exist in both *in vivo* and *in vitro* applications. For example, a significant drawback of liposomal-based delivery systems relates to the construction of liposomes that have sufficient stability and the ability of such liposomes to efficiently release their encapsulated contents to targeted cells and tissues.

With respect to the development of liposomal delivery vehicles for use in delivering nucleic acids, the incorporation of cationic lipids as a component of a liposomal vehicle represents an important advancement. Properties of cationic liposomes, which include for example, their stability, size and surface charge, make them ideal carriers for encapsulating and delivering negatively charged nucleic acids to target cells and tissues. The cationic components (e.g., cationic lipids and/or cationic polymers) that comprise such cationic liposomal vehicles facilitate the interaction between the lipid bilayer of the liposome and the negatively charged nucleic acids, and thereby enhance the encapsulation efficiency of such cationic liposomal vehicles. In part due to their positive surface charge, liposomes prepared comprising cationic lipids (e.g., 1,2-dioleoyl-3-trimethylammonium-propane (DOTAP)) have demonstrated an ability to be efficiently loaded with negatively charged nucleic acids and, the use of cationic liposomal vehicles may facilitate the encapsulation of nucleic acids at concentrations that well exceed those which would be achieved using neutral liposomal vehicles. For example, in certain instances, cationic lipid nanoparticle systems may be characterized as having encapsulation efficiencies approaching 100% when loaded with negatively charged nucleic acids. (See, e.g., Li, et al. Pharm Res., 2007, 24: 438-449.)

Tang et al., Biochemical and Biophysical Research Communications, vol. 242, no. 1, 6 January 1998, pages 141-145 describes a "method of synthesis for disulfide-containing cationic lipids" and describes the synthesis of "the lipid, 1,2-dioleoyl-sn-glycero-3-succinyl-2-hydroxyethyl disulfide ornithine conjugate (DOGSDSO)"

Wetzer et al., Biochemical Journal, vol 356, no. Pt 3, 15 June 2001, pages 747-756 describes "plasmid DNA ... complexed with cationic lipids bearing a disulphide bond".

Many cationic lipids employed to construct such liposomal vehicles however, are generally toxic and accordingly, may be of limited utility, particularly in the quantities necessary to deliver therapeutically effective quantities of their encapsulated contents (e.g., nucleic acids). Further limiting the utility of charged liposomal systems, following their administration to a subject, such charged systems may be rapidly cleared from the systemic circulation and thereby limit their distribution and accumulation in targeted cells and tissues. To overcome the technical challenges associated with the use of cationic liposomal vehicles, the use of multicomponent liposomal delivery systems has been employed. In particular, the preparation of liposomal vehicles using ionizable lipids has been employed as a means of modulating the charge of a liposome in response to the changing pH of an environment (e.g., physiological pH) to which the liposome is exposed. Such ionizable lipids therefore accommodate changes in their surface charge, which may be manipulated, for example, to enhance the encapsulation efficiency of the liposome.

Despite the foregoing limitations, and as a result of their ability to facilitate the delivery of encapsulated materials to target cells, liposomal-based vehicles are an attractive carrier for therapeutic agents and remain subject to continued development efforts. While liposomal-based vehicles that comprise a cationic lipid component have shown promising results with regards to encapsulation and stability, there remains a great need for improved liposomal-based delivery systems.

In contrast to charged liposomal-based vehicles, neutral liposomal vehicles are generally characterized as having relatively improved pharmacokinetic properties. However, in part due to the low encapsulation efficiency observed with neutral liposomes, there have been limited studies performed investigating the use of neutral liposomes to deliver therapeutic agents to target cells. There remains a need for novel lipids that incorporate a multi-functional approach for delivering encapsulated nucleic acids and polynucleotides. Particularly needed are lipid nanoparticles that retain some of the beneficial characteristics of both neutral and charged liposomal delivery systems.

### SUMMARY

Described herein are novel lipids and lipid nanoparticles that comprise such lipids. Also disclosed are methods of using such lipids (e.g., cleavable cationic lipids) as a component of a lipid nanoparticle to facilitate the encapsulation of one or more therapeutic agents (e.g., therapeutic polynucleotides) in such lipid nanoparticles. Methods of preparing neutral lipid nanoparticles that are characterized as having high encapsulation efficiencies (e.g., relative to traditional neutral lipid nanoparticles) are also disclosed herein. The novel methods, lipids and compositions described herein employ a multifunctional strategy to facilitate the encapsulation of one or more therapeutic agents into a neutral lipid nanoparticle and the subsequent transfection of one or more target cells with such lipid nanoparticle.

Disclosed herein are novel lipids and related methods of using such lipids to modulate the properties (e.g., surface charge) of for example, liposomal vehicles (e.g., lipid nanoparticles) into which such novel lipids are incorporated. The present inventions relate to methods of manipulating a lipid nanoparticle comprising at least one lipid having a releasable polar head-group, such method comprising a step of contacting the liposomal vehicle with one or more reducing agents to cause the release of the polar head-group from the at least one lipid. Following release of the polar head-group from the lipid, the liposomal vehicle has an overall neutral surface charge (e.g., a net surface charge or zeta potential of about -2.5 to about +2.5mV).

In particular, the present invention provides a method of encapsulating one or more therapeutic agents in a neutral lipid nanoparticle, such method comprising the steps of: (a) loading a lipid nanoparticle comprising at least one reducible cationic lipid with the one or more therapeutic agents; and (b) contacting the lipid nanoparticle with one or more reducing agents such that the lipid nanoparticle is neutralized;
wherein said reducible cationic lipid has the structure: 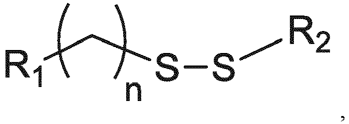
wherein R₁ is a releasable polar head-group and is selected from the group consisting of imidazole, guanidinium, imine, enamine, amino, an optionally-substituted alkyl amino and an optionally-substituted pyridyl;
wherein R₂ is selected from the group consisting of 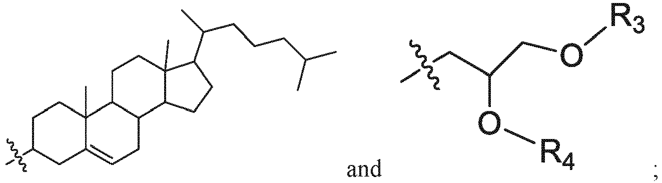
wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl;
wherein n is zero or any positive integer.

In another embodiment, the present invention provides a neutral lipid nanoparticle having one or more encapsulated therapeutic agents prepared in accordance with the method of the present invention.

In another embodiment, the present invention provides a neutral lipid nanoparticle having one or more encapsulated therapeutic agents prepared in accordance with the method of the present invention for use in therapy.

The methods disclosed herein comprise a step of contacting the lipid or lipid nanoparticle with one or more reducing agents such that the polar head-group (R₁) may be cleaved or otherwise liberated from one or more of the cleavable lipids that comprise the lipid nanoparticle (by reduction of the cleavable disulfide linker group), thereby causing the lipophilic R₂ group to remain as a component of the lipid nanoparticle. Cleavage and/or release of the polar R₁ group from the lipid causes the overall charge of the remaining lipid (and lipid nanoparticle which the lipid is a component) to be modified, and causes the lipid nanoparticle to be neutralized.

Contemplated reducing agents include, for example, aqueous solutions comprising one or more *of tris* (2-carboxyethyl)phosphine (TCEP), β-mercaptoethanol (β-ME), dithiothreitol (DTT), glutathione and dithioerythritol. An appropriate reducing agent may be selected based on the nature of the cleavable disulfide linker group that comprises the lipid. For example, a lipid comprising a disulfide linker group that is susceptible to enzymatic digestion may be contacted with or otherwise exposed to an appropriate enzyme to facilitate cleavage of the polar head-group from the lipid. For example, in certain embodiments such enzymes may include one or more of the enzymes selected from the group consisting of thioredoxin and gamma-interferon inducible lysosomal thiol reductase (GILT).

In some embodiments, the degree to which the properties (e.g., surface charge or the average zeta potential) of the lipid nanoparticle are modified is a function of the selected reducing agents to which the lipid nanoparticle is exposed and/or the duration of such exposure. In certain embodiments, the lipid nanoparticle is contacted with the reducing agent from between about five minutes to about twenty four hours (e.g., at least about one minute, two minutes, three minutes, four minutes, five minutes, six minutes, seven minutes, eight minutes, nine minutes, ten minutes, twelve minutes, fifteen minutes, thirty minutes, forty-five minutes, one hour, three hours, six hours, eight hours, twelve hours, sixteen hours, eighteen hours, twenty four hours, or longer). For example, a lipid nanoparticle prepared in accordance with the present invention may have an average zeta potential (Zₐᵥₑ) of at least about +25mV prior to contacting the lipid nanoparticle with the one or more reducing agents. In certain embodiments, the positive charge of the lipid nanoparticle may facilitate encapsulation or loading of one or more therapeutic agents, and in particular negatively charged therapeutic agents (e.g., therapeutic nucleic acids encoding a functional protein or enzyme). For example, the lipid nanoparticles prepared in accordance with the methods disclosed herein may demonstrate high encapsulation efficiencies (e.g., at least 50%, 55%, 60%, 65%, 70%, 75%, 80%, 85%, 87.5%, 90%, 92.5%, 95%, 97.5%, 98%, 99%, 99.5% or more). Similarly, the lipid nanoparticles prepared in accordance with the methods disclosed herein may be characterized with respect to the average concentration of one or more therapeutic agents encapsulated in such compositions. For example, in certain embodiments, the average concentration of therapeutic agent encapsulated in a lipid nanoparticle prepared in accordance with the present inventions is between about 0.025µg/mL to about 250µg/mL (e.g., at least about 0.05µg/mL, 0.1µg/mL, 0.25µg/mL, 0.5µg/mL, 1µg/mL, 2.5µg/mL, 5µg/mL, 10µg/mL, 15µg/mL, 20µg/mL, 25µg/mL, 50µg/mL, 75µg/mL, 100µg/mL, 150µg/mL, 200µg/mL, or more).

Once loaded with the one or more therapeutic agents, the physical properties of the lipid nanoparticles may be modified. For example, the surface charge of a positively charged lipid nanoparticle (e.g., lipid nanoparticles having a Zₐᵥₑ greater than about +20mV) may be reduced or neutralized (e.g., reducing the Zₐᵥₑ to less than about +10mV) based on one or more objectives, such as the characteristics of target cells or tissues. The physical properties of the lipid nanoparticle are modulated by contacting the lipid nanoparticle with an aqueous solution comprising one or more reducing agents. Contacting the lipid nanoparticle with such reducing agents causes one or more of the cleavable disulfide linker groups of the lipid to be cleaved, resulting in the dissociation of the polar head-group (R₁) from the lipophilic group (R₂) of the lipid and a corresponding reduction of the Zₐᵥₑ of the remaining lipid nanoparticle. In certain embodiments, after exposure to a reducing agent the Zₐᵥₑ of the lipid nanoparticle is between about +10mV to about - 20mV (e.g., +10mV, +7.5mV, +5mV, +4mV, +3mV, +2.5mV, +2mV, +1mV, +0.5mV, 0mV, -0.5mV, -1mV, -2mV, -2.5mV, -3mV, -4mV, -5mV, -7.5mV, -10mV, -12.5mV, -15mV or -20mV). In certain embodiments, after exposure to a reducing agent, the Zₐᵥₑ of the lipid nanoparticle is between about -50mV to about -5mV.

The ability to modulate one or more of the physical properties of a lipid nanoparticle (e.g., the ability to reduce the Zₐᵥₑ) relates to the composition of the constituent lipids that comprise such composition, and in particular relates to the presence of one or more cleavable disulfide linker groups that comprise the constituent lipids that comprise such lipid nanoparticle. In certain embodiments, one or more of the constituent lipids that comprise the lipid nanoparticle comprises a cleavable disulfide (S-S) linker group, as represented, by the following structure: 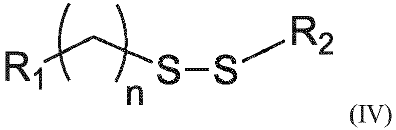 wherein R₁ is a releasable (polar) head-group selected from the group consisting of imidazole, guanidinium, imine, enamine, amino, an optionally-substituted alkyl amino (e.g., an alkyl amino such as dimethylamino) and an optionally-substituted pyridine or pyridyl (e.g., pyridine or nitropyridyl); wherein R₂ is a tail-group selected from the group consisting of: 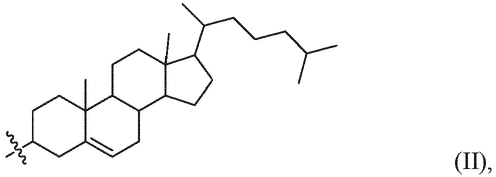 and 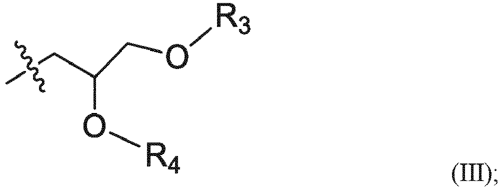 wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl; and wherein n is zero or any positive integer.

Upon contacting the lipid (or a lipid nanoparticle that comprises such lipid) with one or more reducing agents (e.g., an aqueous solution comprising at least one reducing agent capable of reductively cleaving the disulfide linker group), the disulfide linker group is cleaved from the lipid and a polar group represented by the following structure is liberated: 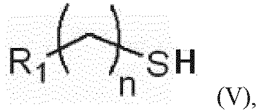 wherein R₁ is the releasable head-group selected from the group consisting of imidazole, guanidinium, imine, enamine, amino, an optionally-substituted alkyl amino (e.g., an alkyl amino such as dimethylamino) and an optionally substituted pyridyl; and the lipid (and the lipid nanoparticle of which the lipid is a component) is thereby neutralized.

In certain embodiments, following exposure of the lipid, and in particular the cleavable disulfide linker group that comprises the lipid, to one or more reducing agents (e.g., β-mercaptoethanol (β-ME)) a thiol group, as represented by the following structure, remains as a component of the lipid nanoparticle: 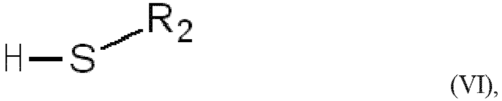 wherein R₂ is selected from the group consisting of: 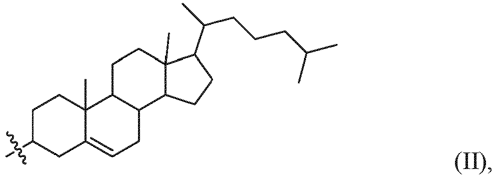 and 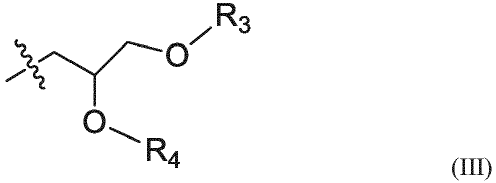 wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl.

In certain embodiments, the cleavable lipids relate to one or more of the lipid compounds and related methods disclosed in co-owned U.S. Application No. 61/494,745 (WO 2012/170889 A1). For example, in some embodiments, the cleavable lipid is the compound 5-(((10,13-dimethyl-17-(6-methylheptan-2-yl)-2,3,4,7,8,9,10,11,12,13,14,15,16,17-tetradecahydro-1H-cyclopenta[a]phenanthren-3-yl)disulfanyl)methyl)-1H-imidazole, having the structure of formula XI (referred to herein as "HGT4001").

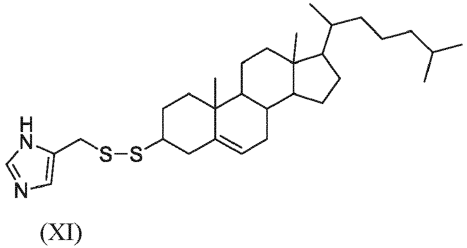

In some embodiments, the cleavable lipid is the compound 1-(2-(((3S,10R,13R)-10,13-dimethyl-17-((R)-6-methylheptan-2-yl)-2,3,4,7,8,9,10,11,12,13,14,15,16,17-tetradecahydro-1H-cyclopenta[a]phenanthren-3 - yl)disulfanyl)ethyl)guanidine, having the structure of formula XII (referred to herein as "HGT4002").

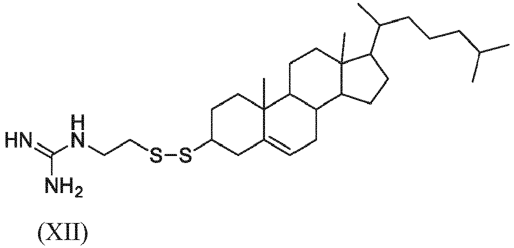

In some embodiments, the cleavable lipid is the compound 2-((2,3-Bis((9Z, 12Z)-octadeca-9, 12-dien-1-yloxy)propyl)disulfanyl)-N,N-dimethylethanamine, having the structure of formula XIII (referred to herein as "HGT4003").

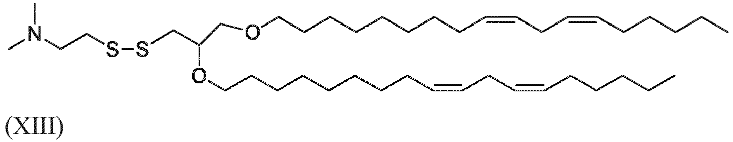

In yet other embodiments, the cleavable lipid is the compound 5-(((2,3-bis((9Z, 12Z)-octadeca-9, 12-dien-1-yloxy)propyl)disulfanyl)methyl)-1H-imidazole having the structure of formula XIV (referred to herein as "HGT4004").

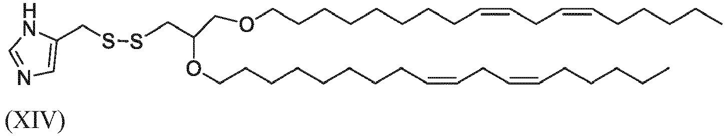

In still other embodiments, the cleavable lipid is the compound 1-(((2,3-bis((9Z,12Z)-octadeca-9,12-dien-1-yloxy)propyl)disulfanyl)methyl)guanidine having the structure of Formula XV (referred to herein as "HGT4005").

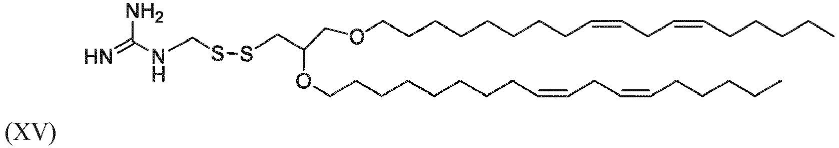

The lipids and lipid nanoparticles described herein may be for use in methods of delivering one or more therapeutic agents to target cells, organs and tissues (e.g., hepatocytes). In certain embodiments, the contemplated therapeutic agents comprise one or more therapeutic nucleic acids or polynucleotides (e.g., DNA or RNA). Accordingly, disclosed herein are methods and compositions for modulating the expression of one or more nucleic acids in a subject. In certain embodiments, such therapeutic nucleic acids comprise or consist of RNA (e.g., mRNA, siRNA, snoRNA or microRNA).

Also disclosed herein are methods of encapsulating one or more therapeutic agents in a neutral lipid nanoparticle. Such methods generally comprise a step of loading the lipid nanoparticle (e.g., a lipid nanoparticle comprising the cleavable lipid HGT4002, DMG-PEG2000, cholesterol and DOPE) with the one or more therapeutic agents and contacting the lipid nanoparticle with one or more reducing agents such that the lipid nanoparticle is neutralized. In a preferred embodiment, the lipid nanoparticle (e.g., a lipid nanoparticle comprising one or more cleavable lipids, a PEG-modified lipid and a helper lipid) prepared in accordance with such methods are loaded with one or more therapeutic agents prior to being contacted with a reducing agent.

In certain embodiments, the lipid compounds disclosed herein are cationic and/or ionizable lipids, that may be used as a lipid nanoparticle or alternatively as component of a lipid nanoparticle. In certain embodiments, the compounds disclosed herein are used to enrich a lipid nanoparticle, thereby conferring improved properties to such enriched lipid nanoparticle (e.g., improved delivery of encapsulated polynucleotides to one or more target cells and/or reduced *in vivo* toxicity of a lipid nanoparticle). Accordingly, also contemplated are lipid nanoparticles, that comprise one or more of the lipids disclosed herein. In certain embodiments, such lipid nanoparticles comprise one or more of a PEG-modified lipid, a non-cationic lipid and a helper lipid.

In certain embodiments one or more of the lipid nanoparticles described herein comprise one or more additional lipids. For example, lipid nanoparticles that comprise or are otherwise enriched with one or more of the cleavable lipid compounds disclosed herein may further comprise one or more of DOTAP (1,2-dioleyl-3-trimethylammonium propane), DODAP (1,2-dioleyl-3-dimethylammonium propane), DOTMA (1,2-di-O-octadecenyl-3-trimethylammonium propane), DLinDMA, DLin-KC2-DMA, C12-200 and ICE. In one embodiment the lipid nanoparticle comprises HGT4001, DOPE and DMG-PEG2000. In another embodiment the lipid nanoparticle comprises HGT4003, DOPE, cholesterol and DMG-PEG2000.

In certain embodiments one or more of the lipid nanoparticles described herein may comprise one or more PEG-modified lipids. For example, lipid nanoparticles that comprise or are otherwise enriched with one or more of the lipid compounds disclosed herein may further comprise one or more of PEG-modified lipids that comprise a poly(ethylene)glycol chain of up to 5kDa in length covalently attached to a lipid comprising one or more C₆-C₂₀ alkyls.

Similarly, the lipid nanoparticles disclosed herein may comprise or may otherwise be enriched with one or more of the lipid compounds disclosed herein and may further comprise one or more of helper lipids that are selected from the group consisting of DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DPPE (1,2-dipalmitoyl-sn-glycero-3-phosphoethanolamine), DMPE (1,2-dimyristoyl-sn-glycero-3-phosphoethanolamine), DOPG (,2-dioleoyl-sn-glycero-3-phospho-(1'-rac-glycerol)), DOPE (1,2-dioleoyl-*sn*-glycero-3-phosphoethanolamine), DSPE (1,2-distearoyl-*sn*-glycero-3-phosphoethanolamine), DLPE (1,2-dilauroyl-*sn*-glycero-3-phosphoethanolamine), DPPS (1,2-dipalmitoyl-*sn-*glycero-3-phospho-L-serine), ceramides, sphingomyelins and cholesterol.

In certain embodiments, the cleavable lipids and the lipid nanoparticles comprising such lipids comprise one or more polynucleotides (e.g., encapsulated DNA or RNA). In other embodiments, the one or more polynucleotides comprise at least one locked nucleic acid (e.g., two, three, four, five, six, seven, eight, nine, ten, twelve, fifteen, sixteen, eighteen, twenty, or more locked nucleic acid residues or monomers). Where the one or more encapsulated polynucleotides comprise RNA, such RNA may include, for example, mRNA, siRNA, snoRNA, microRNA, and combinations thereof.

In certain embodiments, the polynucleotides encapsulated in the lipid nanoparticles hereof comprise mRNA encoding, for example, a functional polypeptide, protein or enzyme, and upon being expressed (i.e., translated) by one or more target cells a functional polypeptide product (e.g., a protein or enzyme) is produced, and in some instances secreted by the target cell into the peripheral circulation of a subject. In certain embodiments, the one or more of the polynucleotides that comprise (or are otherwise loaded or encapsulated into) the lipid nanoparticles described herein encode a nucleic acid (e.g., a polypeptide) which is aberrantly expressed by the subject. In certain embodiments, the one or more of the encapsulated polynucleotides that comprise such lipid nanoparticles encode a functional protein or enzyme such as a urea cycle enzyme (e.g., ornithine transcarbamylase (OTC), carbamoyl-phosphate synthetase 1 (CPS1), argininosuccinate synthetase (ASS1), argininosuccinate lyase (ASL) or arginase 1 (ARG1)). In certain embodiments the one or more of the encapsulated polynucleotides comprises mRNA encoding an enzyme associated with a lysosomal storage disorder (e.g., the encapsulated polynucleotide is mRNA encoding one or more of the enzymes alpha galactosidase A, iduronate-2-sulfatase, iduronate sulfatase, N-acetylglucosamine-1-phosphate transferase, beta-glucosidase and galactocerebrosidase). In other embodiments where the nucleic acids comprise mRNA, such mRNA may encode one or more proteins or enzymes, for example, proteins or enzymes that may be deficient in a subject (e.g., an enzyme or protein selected from the group of enzymes consisting of erythropoietin, human growth hormone, cystic fibrosis transmembrane conductance regulator (CFTR), alpha-L-iduronidase, N-acetylglucosaminidase, alpha-glucosaminide acetyltransferase, N-acetylglucosamine 6-sulfatase, N-acetylgalactosamine-4-sulfatase, galactose-6-sulfate sulfatase, beta-galactosidase, beta-glucuronidase, glucocerebrosidase, heparan sulfamidase, heparin-N-sulfatase, lysosomal acid lipase, acid alpha-glucosidase, arylsulfatase A and hyaluronidase).

Also contemplated herein are lipid nanoparticles that comprise one or more of the lipid compounds disclosed herein and one or more polynucleotides (e.g., antisense oligonucleotides), and in particular polynucleotides that comprises one or more chemical modifications. For example, in certain embodiments where the polynucleotide is mRNA, such chemical modifications render the mRNA more stable and may comprise, for example an end blocking modification of a 5' or 3'untranslated region of the mRNA. In certain embodiments, the chemical modification comprises the inclusion of a partial sequence of a CMV immediate-early 1 (IE1) gene to the 5' untranslated region of the mRNA. In other embodiments the chemical modification comprises the inclusion of a poly A tail to the 3' untranslated region of the mRNA. Also contemplated are chemical modifications that comprise the inclusion of a Cap1 structure to the 5' untranslated region of the mRNA. In still other embodiments, the chemical modification comprises the inclusion of a sequence encoding human growth hormone (hGH) to the 3' untranslated region of the mRNA.

The lipids and lipid nanoparticles described herein may be formulated to specifically target and/or transfect one or more target cells, tissues and organs. In certain embodiments, such lipids and lipid nanoparticles facilitate the transfection of such target cells by one or more mechanisms (e.g., fusogenic-based release and/or proton-sponge mediated disruption of the lipid-bilayer membrane of the target cells). Contemplated target cells include, for example, one or more cells selected from the group consisting of hepatocytes, hematopoietic cells, epithelial cells, endothelial cells, lung cells, bone cells, stem cells, mesenchymal cells, neural cells, cardiac cells, adipocytes, vascular smooth muscle cells, cardiomyocytes, skeletal muscle cells, beta cells, pituitary cells, synovial lining cells, ovarian cells, testicular cells, fibroblasts, B cells, T cells, reticulocytes, leukocytes, granulocytes and tumor cells.

Also disclosed is a neutral lipid nanoparticle having one or more encapsulated therapeutic agents for use in methods of treating disease (e.g., a disease associated with the aberrant expression of a gene or nucleic acid) in a subject. Also contemplated is a neutral lipid nanoparticle having one or more encapsulated therapeutic agents for use in methods of transfecting one or more target cells with one or more polynucleotides, wherein the method comprises contacting the one or more target cells with the lipids or lipid nanoparticles described herein such that the one or more target cells are transfected with the one or more polynucleotides encapsulated therein.

The above discussed and many other features and attendant advantages of the present invention will become better understood by reference to the following detailed description of the invention when taken in conjunction with the accompanying examples. The various embodiments described herein are complimentary and can be combined or used together in a manner understood by the skilled person in view of the teachings contained herein.

### BRIEF DESCRIPTION OF THE DRAWINGS

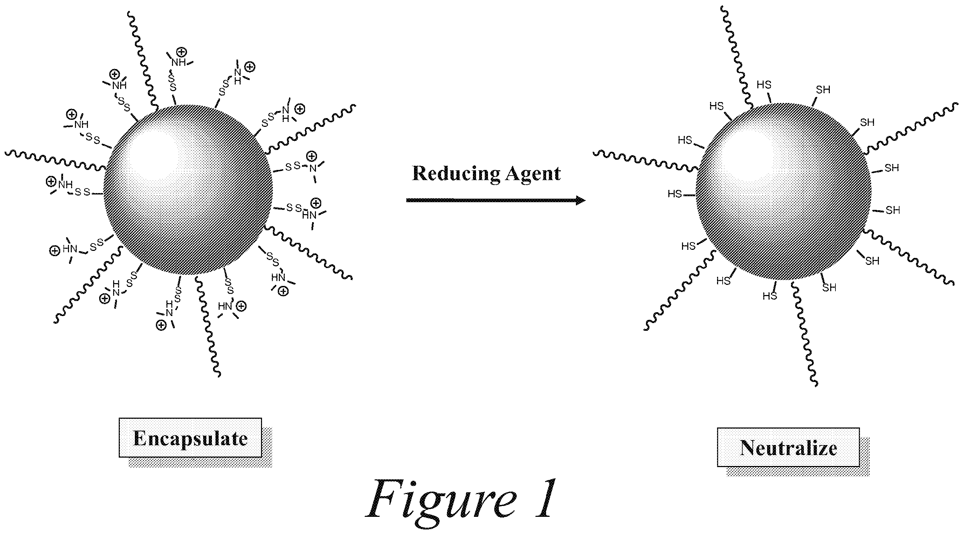

**Figure 1****.** illustrates one embodiment of the present invention whereby a lipid nanoparticle that comprises a cleavable disulfide (S-S) cationic lipid and which is loaded with one or more therapeutic agents is contacted with a reducing agent. As depicted in **FIG. 1****,** prior to contacting the lipid nanoparticle with a reducing agent, the surface of the lipid nanoparticle is positively charged by the ionizable cationic head-groups of the cleavable lipid. Contacting the lipid nanoparticle with the reducing agent causes the disulfide bond (S-S) of the cationic lipid to be cleaved and the cationic amino head-group of the cleavable lipid to dissociate from the lipid nanoparticle. In the depicted embodiment, the surface of the resultant lipid nanoparticle is rendered neutralized.

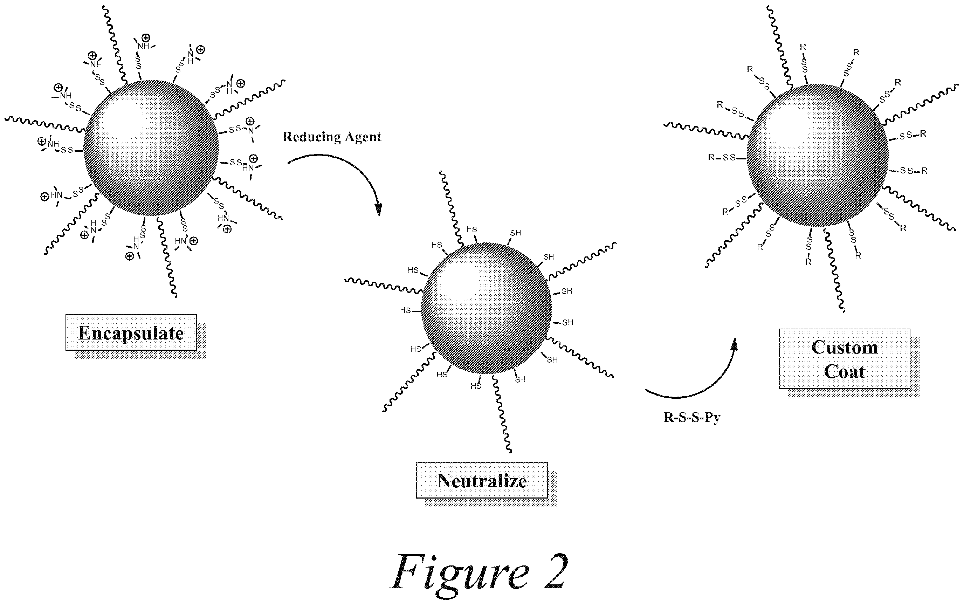

**Figure 2****.** discloses a lipid nanoparticle that comprises a cleavable disulfide (S-S) cationic lipid and which is loaded with one or more therapeutic agents is neutralized by contacting it with a reducing agent. The neutralized lipid is further modified to introduce different functional groups or chemistries. As depicted in **FIG. 2****,** prior to contacting the lipid nanoparticle with a reducing agent, the surface of the lipid nanoparticle is positively charged by the ionizable cationic head-groups of the cleavable lipid. Contacting the lipid nanoparticle with the reducing agent causes the disulfide bond (S-S) of the cationic lipid to be cleaved and the cationic amino head-groups of the cleavable lipid to dissociate from the lipid nanoparticle. The surface of the resultant lipid nanoparticle is rendered neutralized, and as illustrated is coated with sulfhydryl (-SH) functional groups, which may be further reacted to introduce additional functional groups or chemistries. As illustrated in **FIG. 2****,** the neutralized lipid nanoparticle is contacted with a functional R group that is capable of reacting with the sulfhydryl (-SH) functional groups coating the surface of the lipid nanoparticle and thereby binds the functional R group to the surface of the lipid nanoparticle by way of a newly formed disulfide bond.

### DETAILED DESCRIPTION

Provided herein are novel lipids and lipid nanoparticles prepared using such novel lipids. The compositions disclosed herein address many of the limitations associated with traditional surface charge neutral liposomal delivery systems, for example, the poor encapsulation efficiencies that are frequently associated with charge neural lipids. The invention described herein generally relates methods of using novel cleavable cationic lipids to facilitate the efficient encapsulation of one or more therapeutic agents (e.g., polynucleotides) in a lipid nanoparticle.

The pharmacokinetic properties and the ability of a systemically-administered lipid nanoparticle to reach a target cell, organ or tissue are frequently influenced by such lipid nanoparticle's surface charge. For example, following systemic administration to a subject, lipid nanoparticles having a high surface charge are more likely to interact with certain cells, organs or macromolecules in the peripheral circulation, and thereby expedite the excretion of such lipid nanoparticles before they reach the target cells or tissues. As a result, the efficacy and utility of some liposomal-based composition and the therapeutic agents encapsulated therein may be significantly limited.

Lipid nanoparticles that are characterized as being relatively charge neural frequently have demonstrated improved pharmacokinetic characteristics relative to their charged counterparts; however, such charge neutral lipid nanoparticles have been traditionally plagued with poor encapsulation efficiencies, particularly with respect to the encapsulation of nucleic acids or polynucleotides. For example, neutral lipids and surface charge neutral lipid nanoparticles prepared from such lipids demonstrate extremely low encapsulation efficiencies, which are frequently less than 10% with respect to nucleic acid-based therapies. There remains an art-recognized need for lipid nanoparticles that carry a relatively neutral surface charge and that demonstrated high encapsulation efficiencies.

Disclosed herein are novel lipids, lipid nanoparticles and related methods of use that demonstrate improved encapsulation efficiencies, the surface charges of which can be neutralized or otherwise modified. The lipids and related methods described herein therefore retain beneficial properties that are generally associated with charged lipids (e.g., high encapsulation efficiencies), and in certain embodiments can be designed to carry a relatively neutral surface charge.

In general, the lipids disclosed herein comprise a hydrophilic (polar) head-group which is bound to a lipophilic (non-polar) group by way of an intermediate disulfide linker group. Such lipids may be used to prepare lipid nanoparticles suitable for encapsulating one or more therapeutic agents (e.g., polynucleotides encoding a functional protein or enzyme). The disulfide linker group that comprises the lipids can be cleaved by exposure of the lipid to a reducing agent, and the hydrophilic head-group liberated to thereby neutralize the surface charge of the lipid or lipid nanoparticle. The lipids are formulated as a component of a lipid nanoparticle and the surface charge of such composition neutralized after a therapeutic agent has been encapsulated in such composition. Encapsulating the therapeutic agents within the lipid nanoparticle while the surface of such composition is charged improves encapsulation efficiency.

The methods disclosed herein comprise a step of contacting the lipid (or the lipid nanoparticle of which the lipid is a component) with one or more reducing agents such that the polar head-group (R₁) of the lipid may be cleaved or otherwise released from one or more of the cleavable lipids that comprise the lipid nanoparticle (by reduction of the cleavable disulfide linker group), thereby causing the lipophilic R₂ group to remain as a component of the lipid nanoparticle. Cleavage and/or release of the polar head-group (R₁) from the lipid causes the overall charge of the remaining lipid (and the lipid nanoparticle of which the lipid is a component) to be modified, and causes the lipid nanoparticle to be neutralized.

As used herein, the terms "neutral" and "neutralize" refer to a lipid, and in particular a lipid nanoparticle that is substantially surface charge neutral or that is rendered substantially surface charge neutral in accordance with the methods provided herein. The cationic lipids disclosed herein may be used to prepare a lipid nanoparticle having a positive surface charge (e.g., Zₐᵥₑ of about +30mV) and that encapsulates one or more polynucleotides. Such lipid nanoparticle may be modified in accordance with the teachings provided herein such that the composition is rendered relatively neutralized (e.g., Zₐᵥₑ between about -3.0mV and +3.0mV). In certain embodiments, the neutral lipid nanoparticles described herein may be characterized as having a Zₐᵥₑ between about -10.0mV and +10.0mV (e.g., about +10mV, +8mV, +7.5mV, +6mV, +5mV, +4mV, +3mV, +2.5mV, +2mV, +1mV, +0.5mV, +0.25mV, 0mV, -0.25mV, -0.5mV, -1mV, -2mV, -2.5mV, -3mV, -4mV, - 5mV, -7.5mV or -10mV), or preferably between about -2.5mV and +2.5mV. Alternatively, in certain embodiments the modified lipid nanoparticles described herein may be characterized as having a Zₐᵥₑ between about -5mV to about -50mV.

The average zeta potential (Zₐᵥₑ) is indicative of the mean surface charge of a population of lipid nanoparticles and represents a measure of the mean electric charge of such lipid nanoparticle. The Zₐᵥₑ affects particle size, particle stability, encapsulation efficiency and the pharmacokinetic properties of a lipid nanoparticle. In certain embodiments where the therapeutic agent to be encapsulated comprises an anionic polynucleotide (e.g., mRNA encoding a functional enzyme) it may be more efficient to encapsulate such polynucleotide in a lipid nanoparticle that incorporates one or more cationic lipids of the present disclosure. In such an embodiment, the interaction with and encapsulation of the anionic polynucleotide within the cationic lipid bilayer of the lipid nanoparticle serves to enhance the encapsulation efficiency of the lipid nanoparticle. In certain embodiments, once the therapeutic agent has been encapsulated, the surface charge of the lipid nanoparticle may be neutralized in accordance with the teachings hereof.

As used herein, the term "alkyl" refers to both straight and branched chain C₁-C₄₀ hydrocarbons (e.g., C₆-C₂₀ hydrocarbons), and include both saturated and unsaturated hydrocarbons. In certain embodiments, the alkyl may comprise one or more cyclic alkyls and/or one or more heteroatoms such as oxygen, nitrogen, or sulfur and may optionally be substituted with substituents (e.g., one or more of alkyl, halo, alkoxyl, hydroxy, amino, aryl, ether, ester or amide). In certain embodiments, a contemplated alkyl includes (9Z, 12Z)-octadeca-9, 12-dien. The use of designations such as, for example, "C₆-C₂₀" is intended to refer to an alkyl (e.g., straight or branched chain and inclusive of alkenes and alkyls) having the recited range carbon atoms. As used herein, the term "aryl" refers to aromatic groups (e.g., monocyclic, bicyclic and tricyclic structures) containing six to ten carbons in the ring portion. The aryl groups may be optionally substituted through available carbon atoms and in certain embodiments may include one or more heteroatoms such as oxygen, nitrogen or sulfur.

As used herein, the phrase "linker group" refers to an organic functional moiety that is covalently bound to the hydrophilic (polar) head-group and the lipophilic (non-polar) tail-group. In accordance with the present inventions, the linker group can be cleaved by reduction or enzymatically upon being contacted with one or more reducing agents, thereby causing the dissociation of the head-group from the remaining tail-group. It should be noted that the terms "head-group" and "tail-group" as used herein to describe the lipids of the present disclosure, and in particular functional groups that comprise such lipids, are used for ease of reference to describe the orientation of one or more functional groups relative to other functional groups. For example, in certain embodiments a hydrophilic head-group (e.g., guanidinium) is bound (e.g., by one or more of hydrogen-bonds, van der Waals' forces, ionic interactions and covalent bonds) to a cleavable disulfide linker group, which in turn is bound to a hydrophobic tail-group (e.g., cholesterol).

In certain embodiments, at least one of the functional groups or moieties that comprise the lipids disclosed herein is hydrophobic in nature (e.g., a hydrophobic tail-group comprising a naturally-occurring lipid such as cholesterol). As used herein, the term "hydrophobic" is used to indicate in qualitative terms that a functional group is water-avoiding, and typically such groups are not water soluble. For example, disclosed herein are compounds that comprise a cleavable disulfide (S-S) group bound to one or more hydrophobic groups, wherein such hydrophobic groups comprise one or more naturally occurring lipids such as cholesterol, and/or an optionally substituted, variably saturated or unsaturated C₆-C₂₀ alkyl and/or an optionally substituted, variably saturated or unsaturated C₆-C₂₀ acyl.

In certain embodiments, at least one of the functional groups or moieties that comprise the lipids disclosed herein is hydrophilic in nature (e.g., a hydrophilic head-group comprising a cationic imidazole moiety). As used herein, the term "hydrophilic" is used to indicate in qualitative terms that a functional group is water-preferring, and typically such groups are water-soluble. For example, disclosed herein are compounds that comprise a cleavable disulfide (S-S) functional group bound to one or more hydrophilic groups (e.g., a hydrophilic head-group), wherein such hydrophilic groups comprise or are selected from the group consisting of imidazole, guanidinium, amino, imine, enamine, an optionally-substituted alkyl amino (e.g., an alkyl amino such as dimethylamino) and pyridyl. The hydrophilic head-group of the lipid is charged (e.g., a cationic imidazole head-group). Upon cleavage of such a charged linker group from the lipid in accordance with the teachings of the present invention, the hydrophilic head-group bound thereto is liberated from the lipid and the remaining lipid (or the lipid nanoparticle of which such remaining lipid is a component) thereby neutralized.

The present invention relates to a method of neutralizing a lipid nanoparticle such that the surface charge of such composition is modified. Generally, such lipid nanoparticles comprise at least one lipid that has a head-group (e.g., a cationic head-group) that is bound (e.g., covalently bound) to a disulfide linker group. In embodiments, the linker group is (or comprises) a disulfide group that is susceptible to cleavage (e.g., chemical or enzymatic cleavage). Such cleavage may be catalyzed upon exposure to one or more reducing agents. The dissociation of the polar head-group from the lipid (e.g., as may be observed upon cleavage of the disulfide linker group) results in a modification of the charge of such lipid (or the lipid nanoparticle of which such lipid is a component).

The lipid compounds disclosed herein generally comprise one or more cleavable disulfide linker groups. Such linker groups comprise or consist of one or more disulfide (S-S) functional groups as depicted in Formula IV below: 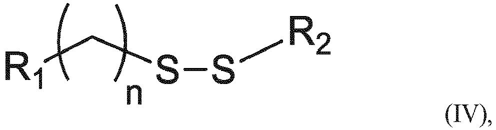 wherein R₁ represents a hydrophilic (polar) head-group and R₂ represents a lipophilic (non-polar) tail-group. In certain embodiments, R₁ may be selected from the group consisting of imidazole, guanidinium, imine, enamine, amino, an optionally-substituted alkyl amino (e.g., an alkyl amino such as dimethylamino) and an optionally-substituted pyridyl. In certain embodiments, R₂ may be selected from the group consisting of: 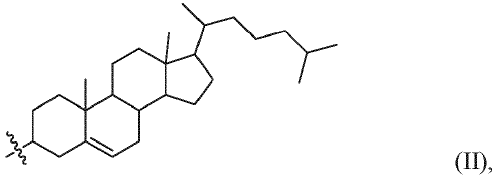 and 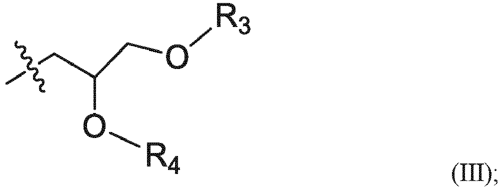 wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl. In certain embodiments, n is 1 (such that the alkyl is ethyl), 2 (such that the alkyl is methyl), 3 (such that the alkyl is, for example, propyl or iso-propyl), 4 (such that the alkyl is, for example, butyl, iso-butyl, sec-butyl or ter-butyl), 5 (such that the alkyl is, for example, pentane), 6 (such that the alkyl is, for example, hexane), 7 (such that the alkyl is, for example, heptane), 8 (such that the alkyl is, for example, octane), 9 (n such that the alkyl is, for example, nonane) or 10 (such that the alkyl is, for example, decane).

As used herein to refer to a lipid or a linker group, the terms "cleave" and "cleavable" generally mean that one or more chemical bonds (e.g., one or more of covalent bonds, hydrogen-bonds, van der Waals' forces and/or ionic interactions) between atoms in or adjacent to the disulfide linker group are broken (reduced) or are capable of being broken upon exposure to a selected reducing agent. In embodiments, the cleavable group is a disulfide functional group, and in particular embodiments is a disulfide group that is capable of being cleaved upon exposure to selected biological conditions (e.g., intracellular conditions). For example, a disulfide function groups may be cleaved enzymatically or by a hydrolysis, or alternatively upon exposure to reducing conditions. Upon cleavage of such disulfide functional group, the one or more functional moieties or groups (e.g., a head-group) that are bound thereto may be liberated. In embodiments, the cleavable group is not an ester group or an ether group.

Enzyme-cleavable groups have been employed for many drug delivery systems and a large portion of such systems have been in the context of a prodrug. (Sherwood, R.F. Adv. Drug Del. Rev. 1996, 22, 269-288). In certain embodiments, such enzymes may also be employed to cleave one or more disulfide groups of the compositions disclosed herein. Exemplary enzymes utilized within the context of prodrugs and that may also be employed to cleave one or more disulfide groups may include, but not be limited to, thioredoxin and gamma-interferon inducible lysosomal thiol reductase (GILT). Further, many enzyme-cleavable groups are related to the specific recognition of a peptide sequence. Exemplary enzyme-cleavable sequences contemplated (and their corresponding enzyme) include, but are not limited to, Val-Cit dipeptide (Cathepsin B) (Toki, et al. J. Org. Chem. 2002, 67: 1866-1872; Yoneda, et al. Bioorg. Med. Chem. Lett. 2008, 18: 1632-1636), GFLG (Cathepsin B) (Nori, et al. Bioconj. Chem. 2003, 14: 44-50; Veronese, et al. Bioconj. Chem, 2005, 16: 775-784), GGGF (Cathepsin B) (DeNardo, et al. Clin. Canc. Res. 2003, 9: 3665s-3972s), PVGLIG (MMP) (Chau, et al. J. Pharm. Sci. 2006, 95: 542-551), AAN (legumain) (Stern et al. Bioconj. Chem. 2009, 20: 500-510).

In certain embodiments, the cleavable disulfide group may be employed to liberate one or more agents from the compositions disclosed herein. For example, contemplated is the employment of one or more agents or cargo (e.g., small molecules, proteins or nucleic acids) upon cleavage of, a disulfide functional group.

The cleavable groups described herein are generally bound (e.g., bound by one or more of hydrogen-bonds, van der Waals' forces, ionic interactions and covalent bonds) to one or more functional moieties or groups (at least one head-group and at least one tail-group). In certain embodiments, at least one of the functional moieties or groups is hydrophilic (a hydrophilic head-group comprising one or more of imidazole, guanidinium, amino, imine, enamine, optionally-substituted alkyl amino and pyridyl). Where the lipid compositions of the present disclosure are incorporated in a lipid nanoparticle, cleavage of the disulfide linker group causes the hydrophilic head-group to dissociate from the lipid while the lipophilic tail group remains as a fixed component of the lipid nanoparticle. For example, as illustrated in FIG. 1 upon contacting a cleavable disulfide (S-S) cationic lipid with a reducing agent, the disulfide bond (S-S) is cleaved and the cationic amino head-group of the cleavable lipid dissociates from the lipid nanoparticle, while the lipophilic tail-group remains as a component of the liposomal vehicle. In the depicted embodiment, the surface of the resultant lipid nanoparticle is rendered neutralized as a result of the cleavage of the disulfide linker group.

The present inventions contemplate cleavage of the disulfide linker group by contacting such disulfide linker group with one or more suitable reducing agents. As used herein, the term "agent" is meant to refer to one or more reagents, compounds or conditions with which (or to which) the lipids of the present disclosure may be contacted and/or interact to catalyze or otherwise induce cleavage of one or more linker groups. In certain embodiments, the agent is a reducing agent that is capable of donating electrons in an oxidation-reduction reaction. Contemplated agents include reducing agents, for example, aqueous solutions comprising one or more of *tris* (2-carboxyethyl)phosphine (TCEP), β-mercaptoethanol (β-ME), dithiothreitol (DTT), glutathione and dithioerythritol. In other embodiments, the reducing agent may comprise one or more enzymes that are capable of enzymatically cleaving a disulfide linker group. Preferably, the selection of a suitable reducing agent is based on the nature of the cleavable disulfide linker group that comprises the lipid and is within the purview of one skilled in the art. For example, a lipid comprising a disulfide linker group that is susceptible to enzymatic digestion may be contacted with or otherwise exposed to an appropriate enzyme to facilitate cleavage of the disulfide linker group and the liberation of the polar head-group from the lipid.

In certain embodiments, a disulfide linker group may be cleavable *in vitro* upon contacting the lipid with an appropriate reducing agent (e.g., an aqueous solution comprising the reducing agent β-mercaptoethanol (β-ME)). For example, a lipid nanoparticle comprising one or more of the cleavable lipids of the present disclosure may be contacted with a reducing agent promptly after loading with one or more therapeutic agents (e.g., polynucleotides encoding a functional protein or enzyme) and thereby neutralize the lipid nanoparticle. Alternatively, a lipid nanoparticle comprising one or more cleavable lipids may be contacted with a reducing agent immediately prior to administration to a subject. Lipid nanoparticles that carry a high surface charge (e.g., Zₐᵥₑ more than about +30mV or less than about - 30mV) are often considered more stable because the particle surface charges serve to repel similarly charged particles, thereby reducing the likelihood that similarly charged particles will aggregate together. By delaying the neutralization of the lipid nanoparticle, such that surface neutralization occurs immediately prior to administration of the lipid nanoparticle to a subject (e.g., within less than six weeks, four weeks, three weeks, two weeks, one week, or less than seventy-two hours, forty-eight hours, twenty-four hours, eighteen hours, twelve hours, nine hours, six hours, three hours, two hours, one hour, thirty minutes, fifteen minutes, ten minutes, five minutes, one minute or less), the stability of lipid nanoparticles may be preserved or otherwise or extended. It should be noted that in certain embodiments, the degree to which the properties (e.g., surface charge) of a lipid nanoparticle are modified is a function of the selected reducing agents to which the lipid nanoparticle is exposed and/or the duration of such exposure.

While in some embodiments described herein, the terms "modified" and "modulated", as they relate to the surface charge of a lipid nanoparticle, refer to the neutralization of a lipid nanoparticle (e.g., modulating the Zₐᵥₑ such that it is between about -2.5mV and +2.5mV), the present disclosures need not be limited to neutralization. Rather, the terms "modified" and "modulated" when used with respect to the surface charge of a lipid nanoparticle are intended to refer broadly to any changes in the physical or chemical properties of such lipid nanoparticle (e.g., modulating a net positive surface charge of a lipid nanoparticle such that the net surface charge is negative). The selected reducing agent and the duration of exposure of the lipid nanoparticle to such reducing agent represent two important variables that can be manipulated by one of skill in the art such that the surface charge of the lipid nanoparticle may be modified or modulated to a desired degree (e.g., Zₐᵥₑ less than about -5mV). Accordingly, in certain embodiments, the lipid nanoparticle is contacted with a selected reducing agent for a selected duration sufficient to achieve the desired result (e.g., reduction in the surface charge by at least about 1-99%). In certain embodiments, a lipid nanoparticle may be contacted with a selected reducing agent for between about five minutes to about seventy-two hours or more (e.g., at least about fifteen minutes, thirty minutes, forty-five minutes, one hour, three hours, six hours, eight hours, twelve hours, sixteen hours, eighteen hours, twenty four hours, forty-eight hours, seventy-two hours, or longer) to achieve one or more desired outcomes or results.

In certain embodiments, there exists a direct relationship between the duration of exposure of the lipid nanoparticle to the selected reducing agent and the resulting change in the surface charge of the lipid nanoparticle. For example, by extending the duration of exposure of a lipid nanoparticle having a net positive surface charge to one or more reducing agents the resulting surface charge of such lipid nanoparticle may be neutralized or reduced to a greater degree (e.g., reducing the average zeta potential to less than about -0.5mV, -1.0mV, -2.5mV, -5.0mV, - 7.0mV, -10.0mV, -12.5mV, -15.0mV, -17.5mV, -20mV, -25mV, -30mV, -40mV, -50mV, or less). In some embodiments, the degree to which the net surface charge of a lipid nanoparticle may be modulated by the methods disclosed herein may also be a function of the properties of the lipid components that comprise such lipid nanoparticle (e.g., one or more helper lipids or PEG-derivatized lipids). Accordingly, the present invention provides means of controlling the surface charge of lipid nanoparticles (and in particular lipid nanoparticles encapsulating one or more therapeutic agents) based on, for example, one or more desired physical properties or targeted organs or tissues. The foregoing is particularly useful for not only facilitating the targeting of lipid nanoparticles to specific cells, tissues or organs, but in certain embodiments may serve to mitigate toxicities associated with some lipid nanoparticles, and in particular cationic lipid nanoparticles. For example, in some embodiments reducing the surface charge of the lipid nanoparticles disclosed herein reduces the toxicities associated with such lipid nanoparticles by minimizing immunogenicity or by minimizing events associated with complement activation.

Upon contacting the lipid with one or more reducing agents (e.g., an aqueous solution comprising at least one reducing agent), the disulfide linker group is cleaved from the lipid and a hydrophilic head-group liberated, leaving the lipophilic (non-polar) tail-group as a component of the lipid nanoparticle. The cleavable lipid comprises a disulfide (S-S) linker group, and upon exposure to a reducing agent (e.g., an aqueous solution of *tris* (2-carboxyethyl)phosphine (TCEP) and/or β-mercaptoethanol (β-ME)), the hydrophilic head-group represented by the following structure is liberated: 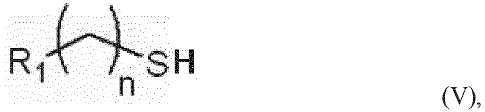 wherein R₁ represents the hydrophilic head-group (e.g., imidazole, guanidinium, imine, enamine, amino, an optionally-substituted alkyl amino, and pyridyl). Similarly, following exposure of the lipid, and in particular a cleavable disulfide linker group that comprises the lipid, to one or more reducing agents, a thiol group, as represented by the following structure, remains as a fixed component of the lipid nanoparticle: 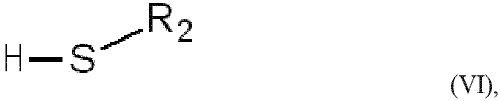 wherein R₂ represents the tail-group and is selected from the group consisting of an optionally-substituted pyridyl (e.g., pyridine or nitropyridyl) or one of the following structures: 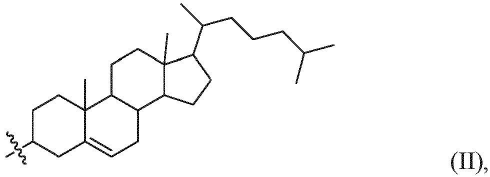 and 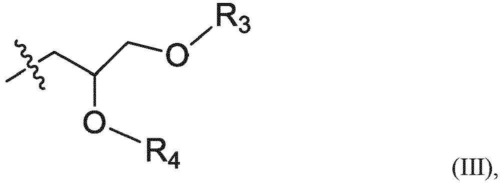 wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl, remains as a component of the lipid nanoparticle. As disclosed in FIG. 2, following exposure of the lipid nanoparticle to a reducing agent, the remaining thiol functional groups that coat the surface of the neutralized composition may be further manipulated. As disclosed in FIG. 2, the thiol groups represent substrates that may be reacted with other organic compounds, polymers, peptides or ligands to further modify the surface of the lipid nanoparticle.

The methods disclosed herein provide means of preparing neutral or slightly charged lipids that demonstrate high encapsulation efficiency. As used herein, the phrase "encapsulation efficiency" refers to the fraction of therapeutic agent that is effectively encapsulated within a liposomal-based vehicle relative to the initial fraction of therapeutic agent present in the lipid phase. In embodiments, the high encapsulations efficiencies are achieved upon loading the lipid nanoparticle (e.g., lipid nanoparticles demonstrating a positive surface charge) prior to contacting such composition with a reducing agent. By loading the liposomal vehicle in its charged state, the encapsulation of oppositely charged therapeutic agents is facilitated. Accordingly, the lipid compounds and liposomal vehicles prepared therefrom are loaded with one or more therapeutic agents prior to being contacted with a reducing agent. In certain embodiments, the lipid compositions and the lipid nanoparticles prepared therefrom exhibit an encapsulation efficiency of at least 50% (e.g., 55%, 60%, 65%, 70%, 75%, 80%, 85%, 87.5%, 90%, 92.5%, 95%, 97.5%, 98%, 99% or more.)

The lipid nanoparticles prepared in accordance with the methods disclosed herein may be characterized with respect to the average concentration of one or more therapeutic agents encapsulated in such compositions. For example, in certain embodiments, the average concentration of therapeutic agent encapsulated in a lipid nanoparticle prepared in accordance with the present inventions is between about 0.025µg/mL to about 250µg/mL (e.g., at least about 0.05µg/mL, 0.1µg/mL, 0.25µg/mL, 0.5µg/mL, 1µg/mL, 2.5µg/mL, 5µg/mL, 10µg/mL, 15µg/mL, 20µg/mL, 25µg/mL, 50µg/mL, 75µg/mL, 100µg/mL, 150µg/mL, 200µg/mL, or more).

In certain embodiments the lipids and the lipid nanoparticles prepared using such lipids are stable. As used herein to characterize a lipid nanoparticle, the term "stable" refers to the physical stability of such compositions and generally means that the composition is suitable for administration to a subject at the conclusion of a predetermined period of time and/or storage conditions (e.g., following storage at standard room temperature and humidity for at least two years). In certain embodiments the substantial absence of a precipitate, cloudiness and/or other particulate matter (e.g., following storage at 45°C for at least two months) in the composition may be indicative of its stability. In other embodiments, the substantial absence of particle aggregation or coalescence may be indicative of the stability of the lipid nanoparticles disclosed herein.

In certain embodiments, the lipid compounds, and in particular the imidazole-based compounds described herein (e.g., HGT4001 and HGT4004), are also characterized by their reduced toxicity, in particular relative to traditional cationic lipids. In some embodiments, the lipid nanoparticles described herein comprise one or more imidazole-based cationic lipid compounds such that the relative concentration of other more toxic cationic lipids in such lipid nanoparticle may be reduced or otherwise eliminated. The imidazole-based compounds or lipids (e.g., HGT4001 and/or HGT4004) may be used as the sole cationic lipid in one or more of the lipid nanoparticles described herein, or alternatively may be combined with traditional cationic lipids (e.g., LIPOFECTIN or LIPOFECTAMINE), non-cationic lipids, PEG-modified lipids and/or helper lipids. In certain embodiments, the lipid nanoparticles may comprise a molar ratio of about 1% to about 90%, about 2% to about 70%, about 5% to about 50%, about 10% to about 40% of the total lipid present in such lipid nanoparticle, or preferably about 20% to about 70% of the total lipid present in such lipid nanoparticle.

The cleavable lipids described herein, and in particular, the disulfide cleavable lipid may consist of one or more of the lipid compounds disclosed in co-owned U.S. Application No. 61/494,745 (WO 2012/170889 A1). For example, in certain embodiments, the invention relates to the compound 5-(((10,13-dimethyl-17-(6-methylheptan-2-yl)-2,3,4,7,8,9,10,11,12,13,14,15,16,17-tetradecahydro-1H-cyclopenta[a]phenanthren-3-yl)disulfanyl)methyl)-1H-imidazole, having the structure of formula XI (referred to herein as "HGT4001").

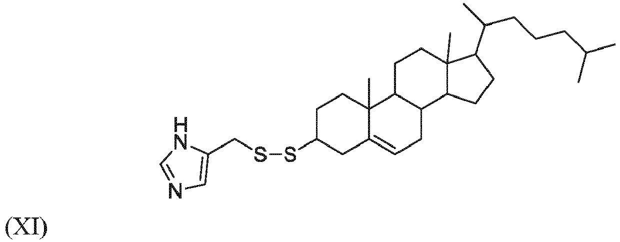

In certain embodiments, the invention relates to the compound 1-(2-(((3S,10R,13R)-10,13-dimethyl-17-((R)-6-methylheptan-2-yl)-2,3,4,7,8,9,10,11,12,13,14,15,16,17-tetradecahydro-1H-cyclopenta[a]phenanthren-3-yl)disulfanyl)ethyl)guanidine, having the structure of formula XII (referred to herein as "HGT4002").

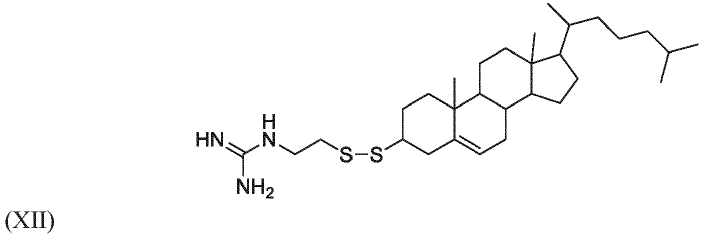

In yet other embodiments, the invention relates to the compound 2-((2,3-Bis((9Z,12Z)-octadeca-9,12-dien-1-yloxy)propyl)disulfanyl)-N,N-dimethylethanamine, having the structure of formula XIII (referred to herein as "HGT4003").

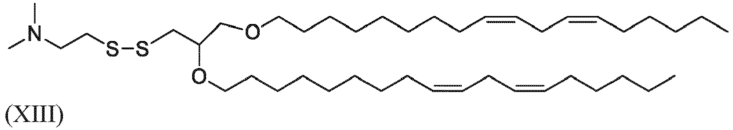

In other embodiments, the invention relates to the compound 5-(((2,3-bis((9Z,12Z)-octadeca-9,12-dien-1-yloxy)propyl)disulfanyl)methyl)-1H-imidazole having the structure of formula XIV (referred to herein as "HGT4004").

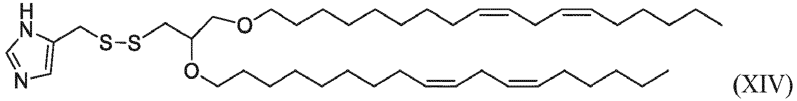

In still other embodiments, invention relates to the compound 1-(((2,3-bis((9Z,12Z)-octadeca-9,12-dien-1-yloxy)propyl)disulfanyl)methyl)guanidine having the structure of formula XV (referred to herein as "HGT4005").

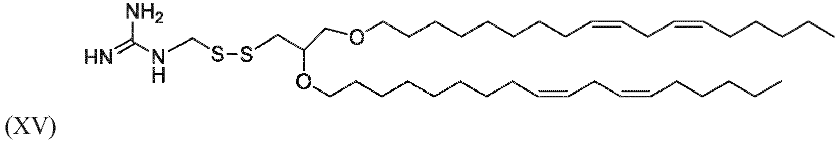

The lipid compounds described herein may be used to construct lipid nanoparticles that facilitate or enhance the delivery and release of encapsulated materials (e.g., one or more therapeutic polynucleotides) to one or more target cells (e.g., by permeating or fusing with the lipid membranes of such target cells). In certain embodiments the compounds described herein are characterized as having one or more properties that afford such compounds advantages relative to other similarly classified lipids. For example, in certain embodiments, the lipids disclosed herein allow for the control and tailoring of the physical properties of lipid nanoparticles of which they are a component. In particular, the surface charge of lipid nanoparticles comprising the lipid compounds disclosed herein may be readily customized based on the teachings provided herein. For example, the surface charge of the lipid nanoparticle is rendered neutral upon exposure to an appropriate reducing agent (e.g., a reducing agent such as TCEP). Such modification may be employed as a means of modulating the pharmacokinetic properties of a lipid nanoparticle (e.g., by enhancing circulatory half-life or by facilitating the distribution of such composition to one or more target cells, organs or tissues). In certain embodiments the lipid compounds and the neutralized lipid nanoparticles disclosed herein exhibit an enhanced (e.g., increased) ability to transfect one or more target cells. As used herein, the terms "transfect" or "transfection" refer to the intracellular introduction of one or more encapsulated materials (e.g., nucleic acids and/or polynucleotides) into a cell, or preferably into a target cell.

The lipids and lipid nanoparticles of the present invention may be for use in methods to facilitate the delivery of a wide range of materials and therapeutic agents to target cells, organs and tissues. Accordingly, the lipids and lipid nanoparticles described herein may be used to encapsulate any number of materials suitable for intracellular delivery. In certain embodiments, such encapsulated materials are capable of conferring a therapeutic or diagnostic benefit upon the cells into which such materials are delivered, and may include any drugs, biologics and/or diagnostics. The materials can be organic or inorganic. Organic molecules can be peptides, proteins, carbohydrates, lipids, sterols, nucleic acids (including peptide nucleic acids), or any combination thereof. In certain embodiments, the lipid nanoparticles described herein can comprise or otherwise encapsulate more than one type of material, for example, two or more different polynucleotide sequences encoding a protein, an enzyme and/or a steroid. In certain embodiments, the encapsulated materials are one or more polynucleotides and nucleic acids.

As used herein, the terms "polynucleotide" and "nucleic acid" are used interchangeably to refer to genetic material (e.g., DNA or RNA), and when such terms are used with respect to the lipid compounds and lipid nanoparticles described herein generally refer to the genetic material encapsulated by such compounds and lipid nanoparticles. In some embodiments, the polynucleotide is RNA. Suitable RNA includes mRNA, siRNA, miRNA, snRNA and snoRNA. Contemplated polynucleotides also include large intergenic non-coding RNA (lincRNA), which generally does not encode proteins, but rather function, for example, in immune signaling, stem cell biology and the development of disease. (*See,* e.g., Guttman, et al., 458: 223-227 (2009); and Ng, et al., Nature Genetics 42: 1035-1036 (2010)). In certain embodiments, the polynucleotides encapsulated by the lipid compounds or lipid nanoparticles in accordance with the invention include RNA or stabilized RNA encoding a protein or enzyme (e.g., mRNA encoding alpha galactosidase A or a low density lipoprotein receptors). The present invention contemplates the use of such polynucleotides (and in particular RNA or stabilized RNA) as a therapeutic that is capable of being expressed by target cells to thereby facilitate the production (and in certain instances the excretion) of a functional enzyme or protein by such target cells as disclosed for example, in International Application No. PCT/US2010/058457 and in United States Provisional Application No. 61/494,881 (WO 2012/170930 A1), filed June 8, 2011. For example, in certain embodiments, upon the expression of one or more polynucleotides by target cells the production of a functional or biologically-active enzyme or protein in which a subject is deficient (e.g., a urea cycle enzyme or an enzyme associated with a lysosomal storage disorder) may be observed.

Also contemplated are the modifications of polynucleotides or nucleic acids. As used herein to describe one or more polynucleotides or nucleic acids described herein (e.g., mRNA), the terms "modification" and "modified", as such terms relate to the polynucleotides or nucleic acids disclosed herein, refer to one or more changes, alterations or substitutions which improve or enhance the stability and/or expression (e.g., translation) of such polynucleotides or nucleic acids (e.g., mRNA), including for example, the inclusion of sequences which function in the initiation of protein translation (e.g., the Kozac consensus sequence). (Kozak, M., Nucleic Acids Res 15 (20): 8125-48 (1987)). In some embodiments, the nucleic acids have undergone a chemical or biological modification to render them more stable. Exemplary modifications to a nucleic acid include the depletion of a base (e.g., by deletion or by the substitution of one nucleotide for another) or modification of a base, for example, the chemical modification of a base. The phrase "chemical modifications" as used herein to describe the nucleic acids or polynucleotides, refers to modifications which introduce chemistries which differ from those seen in naturally occurring nucleic acids, for example, covalent modifications such as the introduction of modified nucleotides, (e.g., nucleotide analogs, or the inclusion of pendant groups which are not naturally found in such nucleic acid molecules). For example, in certain embodiments the polynucleotides disclosed herein (e.g., an mRNA polynucleotide) may comprises at least one modified or chemically modified nucleotide independently selected from the group consisting of 5-methylcytosine, isocytosine, pseudoisocytosine, 5-bromouracil, 5-propynyluracil, 6-aminopurine, 2-aminopurine, inosine, pseudouridine, 2-thiouridine, diaminopurine and 2-chloro-6-aminopurine cytosine.

In addition, suitable polynucleotide modifications include alterations in one or more nucleotides of a codon such that the codon encodes the same amino acid but is more stable than the codon found in the wild-type version of the nucleic acid. For example, an inverse relationship between the stability of RNA and a higher number cytidines (C's) and/or uridines (U's) residues has been demonstrated, and RNA devoid of C and U residues have been found to be stable to most RNases. (Heidenreich, et al. J Biol Chem 269, 2131-8 (1994)). In some embodiments, the number of C and/or U residues in an mRNA sequence is reduced. In a another embodiment, the number of C and/or U residues is reduced by substitution of one codon encoding a particular amino acid for another codon encoding the same or a related amino acid. Contemplated modifications to the mRNA nucleic acids also include the incorporation of pseudouridines. The incorporation of pseudouridines into the mRNA nucleic acids may enhance stability and translational capacity, as well as diminishing immunogenicity *in vivo.* (See, e.g., Kariko, K., et al., Molecular Therapy 16 (11): 1833-1840 (2008)). Substitutions and modifications to the nucleic acids may be performed by methods readily known to one or ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible). For example, the codons for Gly can be altered to GGA or GGG instead of GGU or GGC.

The term modification also includes, for example, the incorporation of non-nucleotide linkages or modified nucleotides into the nucleic acid sequences (e.g., modifications to one or both the 3' and 5' ends of an mRNA molecule encoding a functional protein or enzyme). Such modifications include the addition of bases to a nucleic acid sequence (e.g., the inclusion of a poly A tail or a longer poly A tail), the alteration of the 3' UTR or the 5' UTR, complexing the nucleic acid with an agent (e.g., a protein or a complementary nucleic acid molecule), and inclusion of elements which change the structure of a nucleic acid molecule (e.g., which form secondary structures).

The poly A tail is thought to stabilize natural messengers and synthetic sense RNA. Therefore, in one embodiment a long poly A tail can be added to an mRNA molecule thus rendering the RNA more stable. Poly A tails can be added using a variety of art-recognized techniques. For example, long poly A tails can be added to synthetic or *in vitro* transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)). In one embodiment, the length of the poly A tail is at least about 20, 30, 40, 50, 60, 75, 80, 90, 100, 200, 300, 400, or at least about 500 nucleotides. In one embodiment, the length of the poly A tail is adjusted to control the stability of a modified sense mRNA molecule and, thus, the transcription of protein. For example, since the length of the poly A tail can influence the half-life of a sense mRNA molecule, the length of the poly A tail can be adjusted to modify the level of resistance of the mRNA to nucleases and thereby control the time course of protein expression in a cell. In one embodiment, the stabilized nucleic acid molecules are sufficiently resistant to *in vivo* degradation (e.g., by nucleases), such that they may be delivered to the target cell without a transfer vehicle.

In one embodiment, a nucleic acid encoding a protein can be modified by the incorporation of 3' and/or 5' untranslated (UTR) sequences which are not naturally found in the wild-type nucleic acid. In one embodiment, 3' and/or 5' flanking sequence which naturally flanks an mRNA and encodes a second, unrelated protein can be incorporated into the nucleotide sequence of an mRNA molecule encoding a therapeutic or functional protein in order to modify it. For example, 3' or 5' sequences from mRNA molecules which are stable (e.g., globin, actin, GAPDH, tubulin, histone, or citric acid cycle enzymes) can be incorporated into the 3' and/or 5' region of a sense mRNA nucleic acid molecule to increase the stability of the sense mRNA molecule.

Also contemplated are other modifications to the nucleic acid sequences made to one or both of the 3' and 5' ends of the nucleic acid. For example, the present disclosure contemplates modifications to one or both of the 3' and the 5' ends of the nucleic acids (e.g., mRNA) to include at least a partial sequence of a CMV immediate-early 1 (IE1) gene, or a fragment thereof to improve the nuclease resistance and/or improve the half-life of the nucleic acid. In addition to increasing the stability of the mRNA nucleic acid sequence, it has been surprisingly discovered the inclusion of a partial sequence of a CMV immediate-early 1 (IE1) gene at the 5' end enhances the translation of the mRNA and the expression of the functional protein or enzyme. Also contemplated is the inclusion of a sequence encoding human growth hormone (hGH), or a fragment thereof to one or both of the 3' and 5' ends of the nucleic acid (e.g., mRNA) to further stabilize the nucleic acid. Generally, preferred modifications improve the stability and/or pharmacokinetic properties (e.g., half-life) of the nucleic acid relative to their unmodified counterparts, and include, for example modifications made to improve such nucleic acid's resistance to *in vivo* nuclease digestion.

In certain embodiments, the contemplated polynucleotide modification include changes to the nucleotides to incorporate a sugar moiety having a 2'-substituent group or incorporating a bridged or locked nucleic acid (LNA) structure that can provide increased nuclease resistance. In some embodiments, a preferred modification comprises the inclusion of one or more LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the polynucleotide modifications are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid units as discussed by Christensen, et al., Nucl. Acids. Res. (2002) 30: 4918-4925) and 2'MOE units. In some embodiments there is only one of the above types of modifications present in the oligonucleotide. In some embodiments the polynucleotide comprises only LNA nucleotides and naturally occurring nucleotides (such as RNA), optionally with one or more modified internucleotide linkages such as phosphorothioate.

The nucleotide monomers of the polynucleotides (e.g., mRNA) described herein are coupled together via linkage groups. Suitably, each nucleotide monomer is linked to the 3' adjacent monomer via a linkage group. The person having ordinary skill in the art will understand that, in the context of the present disclosure, the 5' monomer at the end of an oligonucleotide does not comprise a 5' linkage group, although it may or may not comprise a 5' terminal group.

As used herein, the phrases "linkage group" and "internucleotide linkage" are intended to mean a group capable of covalently coupling together two nucleotides. Specific and preferred examples include phosphate groups and phosphorothioate groups. In certain embodiments, the polynucleotides disclosed herein have phosphorothioate internucleotide linkages at each internucleotide linkage. The nucleotide monomers of the polynucleotide or contiguous nucleotides sequence thereof are coupled together via linkage groups. Suitably each nucleotide is linked to the 3' adjacent nucleotide via a linkage group. Suitable internucleotide linkages include those listed within International Application WO 2007/031091, for example the internucleotide linkages listed on the first paragraph of page 34 of WO2007/031091.

In certain embodiments, the lipid compounds and lipid nanoparticles described herein are formulated as a blended formulation or composition. For example, in one embodiment, a lipid nanoparticle comprises a blended formulation comprising a 3:1 ratio of a first lipid nanoparticle comprising HGT4003 and a second lipid nanoparticle comprising HGT4001. Accordingly, also provided herein are blended lipid nanoparticles and related methods for modulating the expression of a polynucleotide in one or more target cells and tissues, as disclosed for example, in United States Provisional Application No. 61/494,714, filed June 8, 2011. Also contemplated are methods for modulating (e.g., increasing or synergistically increasing) the production and/or secretion of, for example, one or more functional polypeptides, proteins or enzymes that are encoded by one or more polynucleotides (e.g., mRNA) encapsulated in such blended lipid nanoparticles by one or more target cells, as also disclosed in United States Provisional Application No. 61/494,714.

In certain embodiments, the lipids and lipid nanoparticles provided herein are capable of modulating the expression of aberrantly expressed nucleic acids and polynucleotides in one or more target cells and tissues. Accordingly, also provided herein are neutral lipid nanoparticles having one or more encapsulated therapeutic agents for use in methods of treating disease in a subject by administering an effective amount of the lipid nanoparticles prepared in accordance with the present invention to the subject. In certain embodiments, such methods may enhance (e.g., increase) the expression of a polynucleotide and/or increase the production and secretion of a functional polypeptide product in one or more target cells and tissues (e.g., hepatocytes). In some embodiments, the targeted cells or tissues aberrantly express the polynucleotide encapsulated by one or more of the lipid compounds or lipid nanoparticles described herein. Also provided herein are neutral lipid nanoparticles having one or more encapsulated therapeutic agents for use in methods of increasing the expression of one or more polynucleotides (e.g., mRNA) in one or more target cells, tissues and organs. Generally, such methods comprise contacting the target cells with one or more lipids and/or lipid nanoparticles that comprise or otherwise encapsulate one or more polynucleotides.

The compounds disclosed herein are used as a lipid (e.g., cationic lipid) component of a lipid nanoparticle. Such lipid nanoparticles are used to encapsulate materials and facilitate the delivery of such materials to one or more target cells, tissues and organs. As used herein, the term "liposome" generally refers to a vesicle composed of lipids (e.g., amphiphilic lipids) arranged in one or more spherical bilayer or bilayers. In embodiments, the liposome is a lipid nanoparticle (e.g., a lipid nanoparticle comprising one or more of the cationic lipid compounds disclosed herein). Such liposomes may be unilamellar or multilamellar vesicles which have a membrane formed from a lipophilic material and an aqueous interior that contains the encapsulated materials (e.g., polynucleotides) to be delivered to one or more target cells, tissues and organs. Examples of suitable lipids (e.g., cationic lipids) that may be used to form the lipid nanoparticles contemplated hereby include one or more of the compounds disclosed herein (e.g., HGT4001, HGT4002, HGT4003, HGT4004 and/or HGT4005). Such lipid nanoparticles may also comprise additional cationic lipids such as C12-200, DLin-KC2-DMA, DOPE, DMG-PEG-2000, non-cationic lipids, cholesterol-based lipids, helper lipids, PEG-modified lipids, as well as the phosphatidyl compounds (e.g., phosphatidylglycerol, phosphatidylcholine, phosphatidylserine, phosphatidylethanolamine, sphingolipids, cerebrosides, and gangliosides) and combinations or mixtures of the forgoing.

Several cationic lipids have been described in the literature, many of which are commercially available. In certain embodiments, such cationic lipids are included in the lipid nanoparticles described herein in addition to one or more of the compounds or lipids disclosed herein (e.g., HGT4003). In some embodiments, the cationic lipid N-[1-(2,3-dioleyloxy)propyl]-N,N,N-trimethylammonium chloride or "DOTMA" is used. (Felgner et al. (Proc. Natl Acad. Sci. 84, 7413 (1987); U.S. Pat. No. 4,897,355). DOTMA can be formulated alone or can be combined with dioleoylphosphatidylethanolamine or "DOPE" or other cationic or non-cationic lipids into a lipid nanoparticle. Other suitable cationic lipids include, for example C12-200, 5-carboxyspermylglycinedioctadecylamide or "DOGS," 2,3-dioleyloxy-N-[2(spermine-carboxamido)ethyl]-N,N-dimethyl-1-propanaminium or "DOSPA" (Behr etal. Proc. Nat.'l Acad. Sci. 86, 6982 (1989); U.S. Pat. No. 5,171,678; U.S. Pat. No. 5,334,761), 1,2-Dioleoyl-3-Dimethylammonium-Propane or "DODAP", 1,2-Dioleoyl-3-Trimethylammonium-Propane or "DOTAP". Contemplated cationic lipids also include 1,2-distearyloxy-N,N-dimethyl-3-aminopropane or "DSDMA", 1,2-dioleyloxy-N,N-dimethyl-3-aminopropane or "DODMA", 1,2-dilinoleyloxy-N,N-dimethyl-3-aminopropane or "DLinDMA", 1,2-dilinolenyloxy-N,N-dimethyl-3-aminopropane or "DLenDMA", N-dioleyl-N,N-dimethylammonium chloride or "DODAC", N,N-distearyl-N,N-dimethylammonium bromide or "DDAB", N-(1,2-dimyristyloxyprop-3-yl)-N,N-dimethyl-N-hydroxyethyl ammonium bromide or "DMRIE", 3-dimethylamino-2-(cholest-5-en-3-beta-oxybutan-4-oxy)-1-(ci s,cis-9,12-octadecadienoxy)propane or "CLinDMA", 2-[5'-(cholest-5-en-3-beta-oxy)-3'-oxapentoxy)-3-dimethy 1-1-(cis,cis-9', 1-2'-octadecadienoxy)propane or "CpLinDMA", N,N-dimethyl-3,4-dioleyloxybenzylamine or "DMOBA", 1,2-N,N'-dioleylcarbamyl-3-dimethylaminopropane or "DOcarbDAP", 2,3-Dilinoleoyloxy-N,N-dimethylpropylamine or "DLinDAP", 1,2-N,N'-Dilinoleylcarbamyl-3-dimethylaminopropane or "DLincarbDAP", 1,2-Dilinoleoylcarbamyl-3-dimethylaminopropane or "DLinCDAP", 2,2-dilinoleyl-4-dimethylaminomethyl-[1,3]-dioxolane or "DLin-K-DMA", 2,2-dilinoleyl-4-dimethylaminoethyl-[1,3]-dioxolane or "DLin-K-XTC2-DMA", or mixtures thereof. (Heyes, J., et al., J Controlled Release 107: 276-287 (2005); Morrissey, DV., et al., Nat. Biotechnol. 23(8): 1003-1007 (2005); PCT Publication WO2005/121348A1). The use of cholesterol-based cationic lipids to formulate the lipid nanoparticles is also contemplated. Such cholesterol-based cationic lipids can be used, either alone or in combination with other cationic or non-cationic lipids. Suitable cholesterol-based cationic lipids include, for example, DC-Chol (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335).

In addition, several reagents are commercially available to enhance transfection efficacy. Suitable examples include LIPOFECTIN (DOTMA:DOPE) (Invitrogen, Carlsbad, Calif), LIPOFECTAMINE (DOSPA:DOPE) (Invitrogen), LIPOFECTAMINE2000. (Invitrogen), FUGENE, TRANSFECTAM (DOGS), and EFFECTENE. Also contemplated are cationic lipids such as the dialkylamino-based, imidazole-based, and guanidinium-based lipids. For example, also contemplated is the use of the cationic lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate or "ICE", as disclosed in International Application No. PCT/US2010/058457.

The use and inclusion of polyethylene glycol (PEG)-modified phospholipids and derivatized lipids such as derivatized cerarmides (PEG-CER), including N-Octanoyl-Sphingosine-1-[Succinyl(Methoxy Polyethylene Glycol)-2000] (C8 PEG-2000 ceramide) in the lipid nanoparticles described herein is also contemplated, preferably in combination with one or more of the compounds and lipids disclosed herein. Contemplated PEG-modified lipids include, but are not limited to, a polyethylene glycol chain of up to 5 kDa in length covalently attached to a lipid with alkyl chain(s) of C₆-C₂₀ length. The addition of such components may prevent complex aggregation and may also provide a means for increasing circulation lifetime and increasing the delivery of the lipid-polynucleotide composition to the target tissues, (Klibanov et al. (1990) FEBS Letters, 268 (1): 235-237), or they may be selected to rapidly exchange out of the formulation *in vivo* (see U.S. Pat. No. 5,885,613). Particularly useful exchangeable lipids are PEG-ceramides having shorter acyl chains (e.g., C14 or C18). The PEG-modified phospholipid and derivatized lipids of the present disclosure may comprise a molar ratio from about 0% to about 20%, about 0.5% to about 20%, about 1% to about 15%, about 4% to about 10%, or about 2% of the total lipid present in a liposomal lipid nanoparticle.

Non-cationic lipids may also be used in one or more of the lipid nanoparticles. Such non-cationic lipids are preferably used in combination with one or more of the compounds and lipids disclosed herein. As used herein, the phrase "non-cationic lipid" refers to any neutral, zwitterionic or anionic lipid. As used herein, the phrase "anionic lipid" refers to any of a number of lipid species that carry a net negative charge at a selected pH, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoylphosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), DLPE (1,2-dilauroyl-*sn*-glycero-3-phosphoethanolamine), DPPS (1,2-dipalmitoyl-sn-glycero-3-phospho-L-serine), 16-O-monomethyl PE, 16-O-dimethyl PE, 18-1-trans PE, 1-stearoyl-2-oleoyl-phosphatidyethanolamine (SOPE), ceramides, sphingomyelins, cholesterol, or a mixture thereof. Such non-cationic lipids may be used alone, but are preferably used in combination with other excipients, for example, one or more of the cationic lipids disclosed herein (e.g., HGT4001, HGT4002, HGT4003, HGT4004 and/or HGT4005). When used in combination with a cationic lipid, the non-cationic lipid may comprise a molar ratio of 5% to about 90%, or preferably about 10 % to about 70% of the total lipid present in the lipid nanoparticle.

Also contemplated is inclusion of polymers in the lipid nanoparticles described herein. Suitable polymers may include, for example, polyacrylates, polyalkycyanoacrylates, polylactide, polylactide-polyglycolide copolymers, polycaprolactones, dextran, albumin, gelatin, alginate, collagen, chitosan, cyclodextrins and polyethylenimine. Such polymers may be used alone, but are preferably used in combination with other excipients, for example, one or more of the cationic lipids disclosed herein (e.g., HGT4001, HGT4002, HGT4003, HGT4004 and/or HGT4005).

In certain embodiments, the lipid nanoparticles are formulated and modified to facilitate the transfection (e.g., of a polynucleotide) of a target cell. In another embodiment, the lipid nanoparticles may be selected, prepared and neutralized to optimize delivery of polynucleotides to a target cell, tissue or organ. For example, if the target cell is a hepatocyte the properties of the lipid nanoparticles (e.g., size, surface charge and/or pH) may be optimized to effectively deliver such lipid nanoparticles to the target cell or organ, reduce immune clearance and/or promote retention in that target organ. Alternatively, if the target tissue is the central nervous system the selection and preparation of the lipid nanoparticles must consider penetration of, and retention within the blood brain barrier and/or the use of alternate means of directly delivering such lipid nanoparticles to such target tissue (e.g., via intracerebrovascular or intrathecal administration). In certain embodiments, the lipid nanoparticles described herein may be combined with a complex that facilitates the transfer of encapsulated materials (e.g., a complex which disrupt or improve the permeability of the blood brain barrier and thereby enhance the transfer of such encapsulated polynucleotides to the target cells). While the lipid nanoparticles described herein can facilitate introduction of encapsulated materials such as one or more polynucleotides into target cells, the addition of polycations (e.g., poly L-lysine and protamine) to, for example one or more of the lipid nanoparticles that comprise the lipid nanoparticles as a copolymer can also facilitate, and in some instances markedly enhance the transfection efficiency of several types of cationic liposomes by 2-28 fold in a number of cell lines both *in vitro* and *in vivo.* (*See,* N.J. Caplen, et al., Gene Ther. 1995; 2: 603; S. Li, et al., Gene Ther. 1997; 4, 891.)

The lipid nanoparticles are prepared to encapsulate one or more materials or therapeutic agents (e.g., polynucleotides). The process of incorporating a desired therapeutic agent (e.g., mRNA) into a liposome or a lipid nanoparticle is referred to herein as or "loading" or "encapsulating" (Lasic, et al., FEBS Lett., 312: 255-258, 1992). The lipid nanoparticle-loaded or -encapsulated materials (e.g., polynucleotides) may be completely or partially located in the interior space of the lipid nanoparticle, within the bilayer membrane of the lipid nanoparticle, or associated with the exterior surface of the lipid nanoparticle.

Loading or encapsulating, for example, a polynucleotide into a lipid nanoparticle may serve to protect the polynucleotide from an environment which may contain enzymes or chemicals (e.g., serum) that degrade such polynucleotides and/or systems or receptors that cause the rapid excretion of such polynucleotides. Accordingly, in some embodiments, the compositions described herein are capable of enhancing the stability of the polynucleotide(s) encapsulated thereby, particularly with respect to the environments into which such polynucleotides will be exposed. Encapsulating materials, such as for example polynucleotides into one or more of the lipid nanoparticles described herein also facilitates the delivery of such polynucleotides into the target cells and tissues. In embodiments, the lipid nanoparticles described herein are loaded with one or more therapeutic agents prior to being neutralized.

In certain embodiments, the lipid nanoparticles described herein are prepared by combining multiple lipid components (e.g., one or more of the lipid compounds disclosed herein) with one or more polymer components. For example, a lipid nanoparticle may be prepared using HGT4003, DOPE, CHOL and DMG-PEG2000. A lipid nanoparticle may be comprised of additional lipid combinations in various ratios, including for example, HGT4001, DOPE and DMG-PEG2000. The selection of cationic lipids, non-cationic lipids and/or PEG-modified lipids which comprise the lipid nanoparticles, as well as the relative molar ratio of such lipids to each other, is based upon the characteristics of the selected lipid(s), the nature of the intended target cells or tissues and the characteristics of the materials or polynucleotides to be delivered by the lipid nanoparticle. Additional considerations include, for example, the saturation of the alkyl chain, as well as the size, surface charge, pH, pKa, fusogenicity and toxicity of the selected lipid(s).

The lipid nanoparticles for use in the present invention can be prepared by various techniques which are presently known in the art. Multi-lamellar vesicles (MLV) may be prepared conventional techniques, for example, by depositing a selected lipid on the inside wall of a suitable container or vessel by dissolving the lipid in an appropriate solvent, and then evaporating the solvent to leave a thin film on the inside of the vessel or by spray drying. An aqueous phase may then added to the vessel with a vortexing motion which results in the formation of MLVs. Unilamellar vesicles (ULV) can then be formed by homogenization, sonication or extrusion of the multi-lamellar vesicles. In addition, unilamellar vesicles can be formed by detergent removal techniques.

During the preparation of lipid nanoparticles described herein, water soluble carrier agents may be also encapsulated in the aqueous interior by including them in the hydrating solution, and lipophilic molecules may be incorporated into the lipid bilayer by inclusion in the lipid formulation. In the case of certain molecules (e.g., cationic or anionic lipophilic polynucleotides), loading of the polynucleotide into preformed lipid nanoparticles or liposomes may be accomplished, for example, by the methods described in U.S. Pat. No. 4,946,683. Following encapsulation of the polynucleotide, the lipid nanoparticles may be processed to remove un-encapsulated mRNA through processes such as gel chromatography, diafiltration or ultrafiltration. For example, if it is desirous to remove externally bound polynucleotide from the surface of the lipid nanoparticles described herein, such lipid nanoparticles may be subject to a Diethylaminoethyl SEPHACEL column.

There are several methods for reducing the size, or "sizing", of the lipid nanoparticles disclosed herein, and any of these methods may generally be employed when sizing is used as part of the invention. The extrusion method is a one method of liposome sizing. (Hope, M J et al. Reduction of Liposome Size and Preparation of Unilamellar Vesicles by Extrusion Techniques. In: Liposome Technology (G. Gregoriadis, Ed.) Vol. 1. p 123 (1993)). The method consists of extruding liposomes through a small-pore polycarbonate membrane or an asymmetric ceramic membrane to reduce liposome sizes to a relatively well-defined size distribution. Typically, the suspension is cycled through the membrane one or more times until the desired liposome size distribution is achieved. The liposomes may be extruded through successively smaller pore membranes to achieve gradual reduction in liposome size.

A variety of alternative methods known in the art are available for sizing of a population of lipid nanoparticles. One such sizing method is described in U.S. Pat. No. 4,737,323. Sonicating a liposome or lipid nanoparticle suspension either by bath or probe sonication produces a progressive size reduction down to small ULV less than about 0.05 microns in diameter. Homogenization is another method that relies on shearing energy to fragment large liposomes into smaller ones. In a typical homogenization procedure, MLV are recirculated through a standard emulsion homogenizer until selected liposome sizes, typically between about 0.1 and 0.5 microns, are observed. The size of the lipid nanoparticles may be determined by quasi-electric light scattering (QELS) as described in Bloomfield, Ann. Rev. Biophys. Bioeng., 10:421-450 (1981). Average lipid nanoparticle diameter may be reduced by sonication of formed lipid nanoparticles. Intermittent sonication cycles may be alternated with QELS assessment to guide efficient liposome synthesis.

Selection of the appropriate size of the lipid nanoparticles described herein must take into consideration the site of the target cell or tissue and to some extent the application for which the lipid nanoparticle is being made. As used herein, the phrase "target cell" refers to cells to which one or more of the lipid nanoparticles described herein are to be directed or targeted. In some embodiments, the target cells comprise a particular tissue or organ. In some embodiments, the target cells are deficient in a protein or enzyme of interest. For example, where it is desired to deliver a polynucleotide to a hepatocyte, the hepatocyte represents the target cell. The lipid nanoparticles (and for example the polynucleotide materials encapsulated therein) may be used to transfect the target cells on a discriminatory basis (i.e., do not transfect non-target cells). The compositions may be prepared to preferentially target a variety of target cells, which include, but are not limited to, hepatocytes, hematopoietic cells, epithelial cells, endothelial cells, lung cells, bone cells, stem cells, mesenchymal cells, neural cells (e.g., meninges, astrocytes, motor neurons, cells of the dorsal root ganglia and anterior horn motor neurons), photoreceptor cells (e.g., rods and cones), retinal pigmented epithelial cells, secretory cells, cardiac cells, adipocytes, vascular smooth muscle cells, cardiomyocytes, skeletal muscle cells, beta cells, pituitary cells, synovial lining cells, ovarian cells, testicular cells, fibroblasts, B cells, T cells, reticulocytes, leukocytes, granulocytes and tumor cells.

Following transfection of one or more target cells by, for example, the polynucleotides encapsulated in the one or more lipid nanoparticles disclosed herein, the production of the product (e.g., a polypeptide or protein) encoded by such polynucleotide may be preferably stimulated and the capability of such target cells to express the polynucleotide and produce, for example, a polypeptide or protein of interest is enhanced. For example, transfection of a target cell by one or more lipid nanoparticles encapsulating mRNA will enhance (i.e., increase) the production of the protein or enzyme encoded by such mRNA.

In some embodiments, it may be desirable to limit transfection of the polynucleotides to certain cells or tissues. For example, the liver represents an important target organ for the compositions disclosed herein in part due to its central role in metabolism and production of proteins and accordingly diseases which are caused by defects in liver-specific gene products (e.g., the urea cycle disorders) may benefit from specific targeting of cells (e.g., hepatocytes). Accordingly, the structural characteristics of the target tissue may be exploited to direct the distribution of the lipid nanoparticles (e.g., an HGT4001-based lipid nanoparticle) to such target tissues. For example, to target hepatocytes one or more of the lipid nanoparticles may be sized or their surface charge modified such that they are able to readily penetrate such endothelial fenestrations to reach the target hepatocytes. Alternatively, a lipid nanoparticle may be sized or its surface charge modified such that the dimensions of the liposome are of a sufficient diameter to limit or expressly avoid distribution into certain cells or tissues. For example, lipid nanoparticles may be sized such that their dimensions are larger than the fenestrations of the endothelial layer lining hepatic sinusoids to thereby limit distribution of the liposomal lipid nanoparticle to hepatocytes. In such an embodiment, large lipid nanoparticles will not easily penetrate the endothelial fenestrations, and would instead be cleared by the macrophage Kupffer cells that line the liver sinusoids. Sizing of, for example, the lipid nanoparticles may therefore provide an opportunity to further manipulate and precisely control the degree to which expression of the encapsulated polynucleotides may be enhanced in one or more target cells. Generally, the size of at least one of the lipid nanoparticles is within the range of about 25 to 250 nm, preferably less than about 250nm, 175nm, 150nm, 125nm, 100nm, 75nm, 50nm, 25nm or 10nm.

Similarly, the compositions may be prepared to preferentially distribute to other target tissues, cells or organs, such as the heart, lungs, kidneys, spleen. For example, the lipid nanoparticles may be prepared to achieve enhanced delivery to the target cells and tissues. Accordingly, the compositions may be enriched with additional cationic, non-cationic and PEG-modified lipids to further target tissues or cells.

In some embodiments, the lipid compounds and the lipid nanoparticles described herein (e.g., HGT4002-based lipid nanoparticles) distribute to the cells and tissues of the liver to enhance the delivery, transfection and the subsequent expression of the polynucleotides (e.g., mRNA) encapsulated therein by the cells and tissues of the liver (e.g., hepatocytes) and the corresponding production of the polypeptide or protein encoded by such polynucleotide. While such compositions may preferentially distribute into the cells and tissues of the liver, the therapeutic effects of the expressed polynucleotides and the subsequent production of a protein encoded thereby need not be limited to the target cells and tissues. For example, the targeted hepatocytes may function as a "reservoir" or "depot" capable of expressing or producing, and systemically or peripherally excreting a functional protein or enzyme, as disclosed for example, in International Application No. PCT/US2010/058457 (WO2011/068810A1) and in United States Provisional Application No. 61/494,881 (WO 2012/170930 A1). It should be understood that the lipid compositions, liposomal vehicles and related methods disclosed herein may be used to target any cells and/or tissues such that those targeted cells and/or tissues function as a reservoir or depot capable of expressing, producing or otherwise secreting a functional enzyme or protein. For example, the compositions disclosed herein may preferentially target and distribute to the cells and tissues of the lung, heart, kidneys, liver and/or spleen such that those cells are rendered capable of expressing (e.g., translating) the functional protein or enzyme that is encoded by the polynucleotide (e.g., an mRNA polynucleotide) encapsulated in the liposomal vehicles (e.g., lipid nanoparticles) disclosed herein. Accordingly, the one or more of the lipid nanoparticles described herein (e.g., HGT4005-based lipid nanoparticles) may target hepatocytes and/or preferentially distribute to the cells and tissues of the liver upon delivery. Following the transfection of the target hepatocytes by the polynucleotide encapsulated in one or more of such lipid nanoparticles, such polynucleotides are expressed (e.g., translated) and a functional product (e.g., a polypeptide or protein) is excreted and systemically distributed, where such functional product may exert a desired therapeutic effect.

The polynucleotides encapsulated in one or more of the lipid compounds or lipid nanoparticles described herein can be delivered to and/or transfect targeted cells or tissues. In some embodiments, the encapsulated polynucleotides are capable of being expressed and functional polypeptide products produced (and in some instances excreted) by the target cell, thereby conferring a beneficial property to, for example the target cells or tissues. Such encapsulated polynucleotides may encode, for example, a hormone, enzyme, receptor, polypeptide, peptide or other protein of interest. In certain embodiments, such encapsulated polynucleotides may also encode a small interfering RNA (siRNA) or antisense RNA for the purpose of modulating or otherwise decreasing or eliminating the expression of an endogenous nucleic acid or gene. In certain embodiments such encapsulated polynucleotides may be natural or recombinant in nature and may exert their therapeutic activity using either sense or antisense mechanisms of action (e.g., by modulating the expression of a target gene or nucleic acid).

In some embodiments, the encapsulated polynucleotides (e.g., mRNA encoding a deficient protein) may optionally include chemical or biological modifications which, for example, improves the stability and/or half-life of such polynucleotide or which improves or otherwise facilitates translation of such polynucleotide.

The co-delivery of one or more unique polynucleotides to target cells by the lipids or lipid nanoparticles described herein is also contemplated, for example, by combining two unique therapeutic agents or polynucleotides into a single lipid nanoparticle. Also contemplated is the delivery of one or more encapsulated polynucleotides to one or more target cells to treat a single disorder or deficiency, wherein each such polynucleotide functions by a different mechanism of action. For example, the lipid nanoparticles may comprise a first polynucleotide which, for example, is encapsulated in a lipid nanoparticle and intended to correct an endogenous protein or enzyme deficiency, and a second polynucleotide intended to deactivate or "knock-down" a malfunctioning endogenous polynucleotide and its protein or enzyme product. Such encapsulated polynucleotides may encode, for example mRNA and siRNA.

While *in vitro* transcribed polynucleotides (e.g., mRNA) may be transfected into target cells, such polynucleotides may be readily and efficiently degraded by the cell *in vivo,* thus rendering such polynucleotides ineffective. Moreover, some polynucleotides are unstable in bodily fluids (particularly human serum) and can be degraded or digested even before reaching a target cell. In addition, within a cell, a natural mRNA can decay with a half-life of between 30 minutes and several days. Accordingly, in certain embodiments, the encapsulated polynucleotides provided herein, and in particular the mRNA polynucleotides provided herein, preferably retain at least some ability to be expressed or translated, to thereby produce a functional protein or enzyme within one or more target cells.

As used herein, the term "subject" refers to any animal (e.g., a mammal), including, but not limited to, humans, non-human primates, rodents, and the like, to which the lipids, compounds or lipid nanoparticles disclosed herein may be administered. Typically, the terms "subject" and "patient" are used interchangeably herein in reference to a human subject.

The ability of the lipids, compounds and/or lipid nanoparticles described herein to modulate or enhance the expression of encapsulated polynucleotides and the production of a polypeptide or protein provides novel and more efficient means of effectuating the *in vivo* production of polypeptides and proteins for the treatment of a host of diseases or pathological conditions. Such lipid nanoparticle compositions are particularly suitable for the treatment of diseases or pathological conditions associated with the aberrant expression of nucleic acids encoding a protein or enzyme. For example, the successful delivery of polynucleotides such as mRNA to target organs such as the liver and in particular, to hepatocytes, can be used for the treatment and the correction of in-born errors of metabolism that are localized to the liver. Accordingly, the lipids, compounds, lipid nanoparticles and related methods described herein may be employed to treat a wide range of diseases and pathological conditions, in particular those diseases which are due to protein or enzyme deficiencies. The polynucleotides encapsulated by the lipids or lipid nanoparticles described herein (e.g., HGT4004-based lipid nanoparticles) may encode a functional product (e.g., a protein, enzyme, polypeptide, peptide, functional RNA, and/or antisense molecule), and preferably encodes a product whose *in vivo* production is desired.

The lipids and lipid nanoparticles disclosed herein are broadly applicable to the delivery of therapeutic agents such as polynucleotides, and in particular mRNA, to treat a number of disorders. In particular, such compounds and compositions are suitable for the treatment of diseases or disorders relating to the deficiency of proteins and/or enzymes. In certain embodiments, the lipid nanoparticle-encapsulated polynucleotides encode functional proteins or enzymes that are excreted or secreted by one or more target cells into the surrounding extracellular fluid (e.g., mRNA encoding hormones and neurotransmitters). Alternatively, in another embodiment, the polynucleotides encapsulated by the lipids and/or lipid nanoparticles encode functional proteins or enzymes that remain in the cytosol of one or more target cells (e.g., mRNA encoding an enzyme associated with urea cycle or lysosomal storage metabolic disorders). Other disorders for which the lipids, compounds and lipid nanoparticles are useful include, but are not limited to, disorders such as SMN1-related spinal muscular atrophy (SMA); amyotrophic lateral sclerosis (ALS); GALT-related galactosemia; Cystic Fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/Ataxia Syndrome and Fragile X Premature Ovarian Failure Syndrome; Prader-Willi syndrome; Fabry disease; hereditary hemorrhagic telangiectasia (AT); Niemann-Pick disease Type C1; the neuronal ceroid lipofuscinoses-related diseases including Juvenile Neuronal Ceroid Lipofuscinosis (JNCL), Juvenile Batten disease, Santavuori-Haltia disease, Jansky-Bielschowsky disease, and PTT-1 and TPP1 deficiencies; EIF2B1, EIF2B2, EIF2B3, EIF2B4 and EIF2B5-related childhood ataxia with central nervous system hypomyelination/vanishing white matter; CACNA1A and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A and SCN1B-related seizure disorders; the Polymerase G-related disorders which include Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; and Wilson's disease. In certain embodiments, the polynucleotides, and in particular mRNA, may encode functional proteins or enzymes. For example, the compositions may include mRNA encoding ornithine transcarbamylase (OTC), carbamoyl-phosphate synthetase 1 (CPS1), argininosuccinate synthetase (ASS1), argininosuccinate lyase (ASL) or arginase 1 (ARG1), cystic fibrosis transmembrane conductance regulator (CFTR), acid alpha glucosidase, arylsulfatase A, alpha galactosidase A, erythropoietin, α1-antitrypsin, carboxypeptidase N, alpha-L-iduronidase, iduronate-2-sulfatase, iduronate sulfatase, N-acetylglucosamine-1-phosphate transferase, N-acetylglucosaminidase, alpha-glucosaminide acetyltransferase, N-acetylglucosamine 6-sulfatase, N-acetylgalactosamine-4-sulfatase, beta-glucosidase, galactose-6-sulfate sulfatase, beta-galactosidase, beta-glucuronidase, glucocerebrosidase, heparan sulfamidase, heparin-N-sulfatase, lysosomal acid lipase, hyaluronidase, galactocerebrosidase, human growth hormone, survival motor neuron, Factor VIII, Factor IX or low density lipoprotein receptors.

In one embodiment, the mRNA encodes a protein or an enzyme selected from the group consisting of human growth hormone, erythropoietin, α1-antitrypsin, acid alpha glucosidase, arylsulfatase A, carboxypeptidase N, α-galactosidase A, alpha-L-iduronidase, iduronate-2-sulfatase, iduronate sulfatase, N-acetylglucosamine-1-phosphate transferase, N-acetylglucosaminidase, alpha-glucosaminide acetyltransferase, N-acetylglucosamine 6-sulfatase, N-acetylgalactosamine-4-sulfatase, beta-glucosidase, galactose-6-sulfate sulfatase, beta-galactosidase, beta-glucuronidase, glucocerebrosidase, heparan sulfamidase, heparin-N-sulfatase, lysosomal acid lipase, hyaluronidase, galactocerebrosidase, ornithine transcarbamylase (OTC), carbamoyl-phosphate synthetase 1 (CPS1), argininosuccinate synthetase (ASS1), argininosuccinate lyase (ASL), arginase 1 (ARG1), cystic fibrosis transmembrane conductance regulator (CFTR), survival motor neuron (SMN), Factor VIII, Factor IX and low density lipoprotein receptors (LDLR).

The lipids, compounds and lipid nanoparticles described herein may be administered to a subject. In some embodiments, the compositions are formulated in combination with one or more additional polynucleotides, carriers, targeting ligands or stabilizing reagents or other suitable excipients. Techniques for formulation and administration of drugs may be found in "Remington's Pharmaceutical Sciences," Mack Publishing Co., Easton, Pa., latest edition.

The lipids and lipid nanoparticles may be administered and dosed in accordance with current medical practice, taking into account the clinical condition of the subject, the nature of the encapsulated materials, the site and method of administration, the scheduling of administration, the subject's age, sex, body weight and other factors relevant to clinicians of ordinary skill in the art. The "effective amount" for the purposes herein may be determined by such relevant considerations as are known to those of ordinary skill in experimental clinical research, pharmacological, clinical and medical arts. In some embodiments, the amount administered is effective to achieve at least some stabilization, improvement or elimination of symptoms and other indicators as are selected as appropriate measures of disease progress, regression or improvement by those of skill in the art. For example, a suitable amount and dosing regimen is one that causes at least transient expression of the one or more polynucleotides in the target cells.

Suitable routes of administration of the lipids, compounds and lipid nanoparticles disclosed herein include, for example, oral, rectal, vaginal, transmucosal, or intestinal administration; parenteral delivery, including intramuscular, subcutaneous, intramedullary injections, as well as intrathecal, intracerebroventricular, direct intraventricular, intravenous, intraperitoneal, intranasal, or intraocular injections or infusions. In certain embodiments, the administration of the lipid nanoparticle described herein to a subject facilitates the contacting of such compositions to one or more target cells, tissues or organs.

Alternately, the lipids, compounds and lipid nanoparticles may be administered in a local rather than systemic manner, for example, via injection or infusion of the lipid nanoparticles directly into a targeted tissue, preferably in a depot or sustained release formulation, such that the contacting of the targeted cells with the constituent lipid nanoparticles may be further facilitated. Local delivery can be affected in various ways, depending on the tissue to be targeted. For example, aerosols containing compositions can be inhaled (for nasal, tracheal, or bronchial delivery); compositions can be injected into the site of injury, disease manifestation, or pain, for example; compositions can be provided in lozenges for oral, tracheal, or esophageal application; can be supplied in liquid, tablet or capsule form for administration to the stomach or intestines, can be supplied in suppository form for rectal or vaginal application; or can even be delivered to the eye by use of creams, drops, or even injection. Formulations containing the lipid compounds complexed with therapeutic molecules or ligands can even be surgically administered, for example in association with a polymer or other structure or substance that can allow the compositions to diffuse from the site of implantation to surrounding cells. Alternatively, such compositions can be applied surgically without the use of polymers or supports.

The lipids and lipid nanoparticles (lipid nanoparticles that have been neutralized in accordance with the teachings provided herein) are preferably stable (e.g., when stored under refrigerated conditions for at least one year). In certain embodiments, the stability of the lipids and lipid nanoparticles provided herein may be extended by lyophilizing such compositions. Accordingly, also contemplated herein are lyophilized lipid nanoparticles comprising one or more of the lipid compounds disclosed herein and related methods for the use of such lyophilized compositions as disclosed for example, in United States Provisional Application No. 61/494,882 (WO 2012/170889 A1), filed June 8, 2011.

In certain embodiments, the compositions are formulated such that they are suitable for extended-release of the, for example, polynucleotides or nucleic acids encapsulated therein. Such extended-release compositions may be conveniently administered to a subject at extended dosing intervals. For example, in certain embodiments, the compositions are administered to a subject twice day, daily or every other day. In a certain embodiments, the compositions are administered to a subject twice a week, once a week, every ten days, every two weeks, every three weeks, or more preferably every four weeks, once a month, every six weeks, every eight weeks, every other month, every three months, every four months, every six months, every eight months, every nine months or annually. Also contemplated are compositions and lipid nanoparticles which are formulated for depot administration (e.g., intramuscularly, subcutaneously, intravitreally) to either deliver or release a polynucleotide (e.g., mRNA) over extended periods of time. Preferably, the extended-release means employed are combined with modifications (e.g., chemical modifications) introduced into the polynucleotides to enhance stability.

While certain methods of the present invention have been described with specificity in accordance with certain embodiments, the following examples serve only to illustrate the invention and are not intended to limit the same.

The articles "a" and "an" as used herein in the specification and in the claims, unless clearly indicated to the contrary, should be understood to include the plural referents. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein.

### EXAMPLES

### Example 1

The present example illustrates that HGT4002-based lipid nanoparticles may be reductively neutralized after having been loaded with mRNA polynucleotide constructs. The HGT4002-based lipid nanoparticles were formed via standard ethanol injection methods as described below. (Ponsa, et al., Int. J. Pharm. (1993) 95: 51-56.) Ethanolic stock solutions of the lipids were prepared ahead of time at a concentration of 50 mg/mL and stored at -20°C.

Codon-optimized firefly luciferase (CO-FFL) mRNA was synthesized by *in vitro* transcription from a plasmid DNA template, which was followed by the addition of a 5' cap structure (Cap1) (Fechter, P. et al., J. Gen. Virology (2005) 86: 1239-1249) and a 3' poly(A) tail of approximately 200 nucleotides in length as determined by gel electrophoresis. The 5' and 3' untranslated regions present in each mRNA product are represented as X and Y, respectively in SEQ ID NO: 1, as indicated below.
*Codon-Optimized Firefly Luciferase (FFL) mRNA:* SEQ ID NO: 1 
XAUGGAAGAUGCCAAAAACAUUAAGAAGGGCCCAGCGCCAUUCUACCCACUCGAAGACGGGA
CCGCCGGCGAGCAGCUGCACAAAGCCAUGAAGCGCUACGCCCUGGUGCCCGGCACCAUCGCC
UUUACCGACGCACAUAUCGAGGUGGACAUUACCUACGCCGAGUACUUCGAGAUGAGCGUUCG
GCUGGCAGAAGCUAUGAAGCGCUAUGGGCUGAAUACAAACCAUCGGAUCGUGGUGUGCAGCG
AGAAUAGCUUGCAGUUCUUCAUGCCCGUGUUGGGUGCCCUGUUCAUCGGUGUGGCUGUGGCC
CCAGCUAACGACAUCUACAACGAGCGCGAGCUGCUGAACAGCAUGGGCAUCAGCCAGCCCAC
CGUCGUAUUCGUGAGCAAGAAAGGGCUGCAAAAGAUCCUCAACGUGCAAAAGAAGCUACCGA
UCAUACAAAAGAUCAUCAUCAUGGAUAGCAAGACCGACUACCAGGGCUUCCAAAGCAUGUAC
ACCUUCGUGACUUCCCAUUUGCCACCCGGCUUCAACGAGUACGACUUCGUGCCCGAGAGCUU
CGACCGGGACAAAACCAUCGCCCUGAUCAUGAACAGUAGUGGCAGUACCGGAUUGCCCAAGG
GCGUAGCCCUACCGCACCGCACCGCUUGUGUCCGAUUCAGUCAUGCCCGCGACCCCAUCUUC
GGCAACCAGAUCAUCCCCGACACCGCUAUCCUCAGCGUGGUGCCAUUUCACCACGGCUUCGG
CAUGUUCACCACGCUGGGCUACUUGAUCUGCGGCUUUCGGGUCGUGCUCAUGUACCGCUUCG
AGGAGGAGCUAUUCUUGCGCAGCUUGCAAGACUAUAAGAUUCAAUCUGCCCUGCUGGUGCCC
ACACUAUUUAGCUUCUUCGCUAAGAGCACUCUCAUCGACAAGUACGACCUAAGCAACUUGCA
CGAGAUCGCCAGCGGCGGGGCGCCGCUCAGCAAGGAGGUAGGUGAGGCCGUGGCCAAACGCU
 
UCCACCUACCAGGCAUCCGCCAGGGCUACGGCCUGACAGAAACAACCAGCGCCAUUCUGAUC
ACCCCCGAAGGGGACGACAAGCCUGGCGCAGUAGGCAAGGUGGUGCCCUUCUUCGAGGCUAA
GGUGGUGGACUUGGACACCGGUAAGACACUGGGUGUGAACCAGCGCGGCGAGCUGUGCGUCC
GUGGCCCCAUGAUCAUGAGCGGCUACGUUAACAACCCCGAGGCUACAAACGCUCUCAUCGAC
AAGGACGGCUGGCUGCACAGCGGCGACAUCGCCUACUGGGACGAGGACGAGCACUUCUUCAU
CGUGGACCGGCUGAAGAGCCUGAUCAAAUACAAGGGCUACCAGGUAGCCCCAGCCGAACUGG
AGAGCAUCCUGCUGCAACACCCCAACAUCUUCGACGCCGGGGUCGCCGGCCUGCCCGACGAC
GAUGCCGGCGAGCUGCCCGCCGCAGUCGUCGUGCUGGAACACGGUAAAACCAUGACCGAGAA
GGAGAUCGUGGACUAUGUGGCCAGCCAGGUUACAACCGCCAAGAAGCUGCGCGGUGGUGUUG
UGUUCGUGGACGAGGUGCCUAAAGGACUGACCGGCAAGUUGGACGCCCGCAAGAUCCGCGAG
AUUCUCAUUAAGGCCAAGAAGGGCGGCAAGAUCGCCGUGUAY
**X** = GGGAUCCUACC **(SEQ ID NO: 2)**
**Y** = UUUGAAUU **(SEQ ID NO: 3)**

Next, 0.25mg of the FFL mRNA was denatured by heating it at 70°C for 5 minutes using a heat block. A 3mL volume of an aqueous buffered solution (10 mM citrate/150 mM NaCl, pH 4.5) was prepared and heated in a 15mL RNase-Free conical tube by placing the tube in a heated water bath, and into which was followed by the instant addition of the FFL mRNA into the heated aqueous buffered solution to achieve a final FFL mRNA concentration of 0.25mg/3mL.

All mRNA concentrations were determined via the Ribogreen assay (Invitrogen). Encapsulation of mRNA was calculated by performing the Ribogreen assay with and without the presence of 0.1% Triton-X 100. Particle sizes (dynamic light scattering (DLS)) and zeta potentials were determined using a Malvern Zetasizer instrument in 1x PBS and 1mM KCl solutions, respectively.

To prepare the lipid nanoparticles, aliquots of 50 mg/mL ethanolic solutions of HGT4002, DOPE, cholesterol and DMG-PEG2000 were mixed as indicated below in Table 1, heated to ensure dissolution and diluted with ethanol. The resulting lipid solution is suitable for 0.25mg mRNA at N/P ratio of 4.

**Table 1**

| **Lipid Components** | **Molar Ratio** | **MW** | **µmol** | **Weight (mg)** | **Volume (µL)** |
|---|---|---|---|---|---|
| DMG-PEG2000 | 6 | 2509.19 | 1.04 | 2.6 | 52.2 |
| HGT4002 | 18 | 506 | 3.12 | 1.6 | 31.6 |
| CHOL | 20 | 386.65 | 3.47 | 1.3 | 26.8 |
| DOPE | 56 | 744.03 | 9.71 | 7.2 | 144.4 |
| *Total* | *100* | -- | *17.33* | *12.8* | *255.0* |

0.5mL of the ethanolic lipid solution was injected rapidly into the heated (70°C) aqueous mRNA/buffer solution, shaken and the resulting suspension quickly returned to the 70°C water bath. The resulting nanoparticle suspension was concentrated, re-suspended in a sodium borate buffer (pH 8.0), passed through MUSTANG Q membranes (Pall Life Sciences), was filtered and diafiltrated with 1x PBS (pH 7.4) and further concentrated. The Zₐᵥₑ of the resulting lipid nanoparticles was 159.4nm (Dv₍₅₀₎ = 147nm; Dv₍₉₀₎ = 299nm) and the average Zeta potential was 28.0mV. The lipid nanoparticles demonstrated an encapsulation efficiency of approximately 82%.

The lipid nanoparticles were then exposed to aqueous solutions comprising the reducing agent β-mercaptoethanol (β-ME) and the Zeta potential of the lipid nanoparticle assessed. Exposure of the lipid nanoparticles to 250µL (100X) of a β-ME solution for one hour caused the Zeta potential of the lipid nanoparticle to be reduced to 15.8mV. Exposure of the lipid nanoparticles to 250µL (100X) of a β-ME solution for three hours caused the Zeta potential of the lipid nanoparticle to be reduced to -0.26mV. Exposure of the lipid nanoparticles to 25µL (10X) of a β-ME solution for twenty two hours caused the Zeta potential of the lipid nanoparticle to be reduced to 1.59mV.

The foregoing supports the conclusion that upon exposure to a reducing agent, the charge (i.e., Zeta potential) of HGT4002-based lipid nanoparticles can be modulated, and in certain instances neutralized.

### Example 2

The present example illustrates that HGT4002-based lipid nanoparticles may be reductively neutralized after having been loaded with mRNA polynucleotide constructs. The HGT4002-based lipid nanoparticles were formed via standard ethanol injection methods as described below. (Ponsa, et al., Int. J. Pharm. (1993) 95: 51-56.) Ethanolic stock solutions of the lipids were prepared ahead of time at a concentration of 50 mg/mL and stored at -20°C.

Codon-optimized firefly luciferase (CO-FFL) mRNA was synthesized by *in vitro* transcription from a plasmid DNA template, which was followed by the addition of a 5' cap structure (Cap1) (Fechter, P. et al., J. Gen. Virology (2005) 86: 1239-1249) and a 3' poly(A) tail of approximately 200 nucleotides in length as determined by gel electrophoresis. The 5' and 3' untranslated regions present in each mRNA product are represented as X and Y, respectively in SEQ ID NO: 1, as indicated below.
*Codon-Optimized Firefly Luciferase (FFL) mRNA:* **SEQ ID NO: 1** 
XAUGGAAGAUGCCAAAAACAUUAAGAAGGGCCCAGCGCCAUUCUACCCACUCGAAGACGGGA
CCGCCGGCGAGCAGCUGCACAAAGCCAUGAAGCGCUACGCCCUGGUGCCCGGCACCAUCGCC
UUUACCGACGCACAUAUCGAGGUGGACAUUACCUACGCCGAGUACUUCGAGAUGAGCGUUCG
GCUGGCAGAAGCUAUGAAGCGCUAUGGGCUGAAUACAAACCAUCGGAUCGUGGUGUGCAGCG
AGAAUAGCUUGCAGUUCUUCAUGCCCGUGUUGGGUGCCCUGUUCAUCGGUGUGGCUGUGGCC
CCAGCUAACGACAUCUACAACGAGCGCGAGCUGCUGAACAGCAUGGGCAUCAGCCAGCCCAC
CGUCGUAUUCGUGAGCAAGAAAGGGCUGCAAAAGAUCCUCAACGUGCAAAAGAAGCUACCGA
UCAUACAAAAGAUCAUCAUCAUGGAUAGCAAGACCGACUACCAGGGCUUCCAAAGCAUGUAC
ACCUUCGUGACUUCCCAUUUGCCACCCGGCUUCAACGAGUACGACUUCGUGCCCGAGAGCUU
CGACCGGGACAAAACCAUCGCCCUGAUCAUGAACAGUAGUGGCAGUACCGGAUUGCCCAAGG
GCGUAGCCCUACCGCACCGCACCGCUUGUGUCCGAUUCAGUCAUGCCCGCGACCCCAUCUUC
GGCAACCAGAUCAUCCCCGACACCGCUAUCCUCAGCGUGGUGCCAUUUCACCACGGCUUCGG
CAUGUUCACCACGCUGGGCUACUUGAUCUGCGGCUUUCGGGUCGUGCUCAUGUACCGCUUCG
AGGAGGAGCUAUUCUUGCGCAGCUUGCAAGACUAUAAGAUUCAAUCUGCCCUGCUGGUGCCC
ACACUAUUUAGCUUCUUCGCUAAGAGCACUCUCAUCGACAAGUACGACCUAAGCAACUUGCA
CGAGAUCGCCAGCGGCGGGGCGCCGCUCAGCAAGGAGGUAGGUGAGGCCGUGGCCAAACGCU
UCCACCUACCAGGCAUCCGCCAGGGCUACGGCCUGACAGAAACAACCAGCGCCAUUCUGAUC
ACCCCCGAAGGGGACGACAAGCCUGGCGCAGUAGGCAAGGUGGUGCCCUUCUUCGAGGCUAA
GGUGGUGGACUUGGACACCGGUAAGACACUGGGUGUGAACCAGCGCGGCGAGCUGUGCGUCC
GUGGCCCCAUGAUCAUGAGCGGCUACGUUAACAACCCCGAGGCUACAAACGCUCUCAUCGAC
AAGGACGGCUGGCUGCACAGCGGCGACAUCGCCUACUGGGACGAGGACGAGCACUUCUUCAU
CGUGGACCGGCUGAAGAGCCUGAUCAAAUACAAGGGCUACCAGGUAGCCCCAGCCGAACUGG
AGAGCAUCCUGCUGCAACACCCCAACAUCUUCGACGCCGGGGUCGCCGGCCUGCCCGACGAC
GAUGCCGGCGAGCUGCCCGCCGCAGUCGUCGUGCUGGAACACGGUAAAACCAUGACCGAGAA
GGAGAUCGUGGACUAUGUGGCCAGCCAGGUUACAACCGCCAAGAAGCUGCGCGGUGGUGUUG
UGUUCGUGGACGAGGUGCCUAAAGGACUGACCGGCAAGUUGGACGCCCGCAAGAUCCGCGAG
AUUCUCAUUAAGGCCAAGAAGGGCGGCAAGAUCGCCGUGUAY
**X** = GGGAUCCUACC **(SEQ ID NO: 2)**
**Y** = UUUGAAUU **(SEQ ID NO: 3)**

Next, 0.25mg of the FFL mRNA was denatured by heating it at 70°C for 5 minutes using a heat block. A 2mL volume of an aqueous buffered solution (10 mM citrate/150 mM NaCl, pH 4.5) was prepared and heated in an RNase-Free conical tube by placing the tube in a heated water bath, and into which was followed by the instant addition of the FFL mRNA into the heated aqueous buffered solution to achieve a final FFL mRNA concentration of 0.25mg/2mL.

All mRNA concentrations were determined via the Ribogreen assay (Invitrogen). Encapsulation of mRNA was calculated by performing the Ribogreen assay with and without the presence of 0.1% Triton-X 100. Particle sizes (dynamic light scattering (DLS)) and zeta potentials were determined using a Malvern Zetasizer instrument in 1x PBS and 1mM KCl solutions, respectively.

To prepare the lipid nanoparticles, aliquots of 50 mg/mL ethanolic solutions of HGT4002, DOPE, cholesterol and DMG-PEG2000 were mixed as indicated in Table 2 below, heated to ensure dissolution and diluted with ethanol. The resulting lipid solution is suitable for 0.25mg mRNA at N/P ratio of 4.

**Table 2**

| **Lipid Components** | **Molar Ratio** | **MW** | **µmol** | **Weight (mg)** | **Volume (µL)** |
|---|---|---|---|---|---|
| DMG-PEG2000 | 6 | 2509.19 | 1.04 | 2.6 | 52.2 |
| HGT4002 | 18 | 506 | 3.12 | 1.6 | 31.6 |
| CHOL | 20 | 386.65 | 3.47 | 1.3 | 26.8 |
| DOPE | 56 | 744.03 | 9.71 | 7.2 | 144.4 |
| *Total* | *100* | -- | *17.33* | *12.8* | *255.0* |

0.5mL of the ethanolic lipid solution was injected rapidly into the heated (70°C) aqueous mRNA/buffer solution, shaken and the resulting suspension quickly returned to the 70°C water bath. The resulting nanoparticle suspension was concentrated, re-suspended in a sodium borate buffer (pH 8.0), passed through MUSTANG Q membranes (Pall Life Sciences), was filtered and diafiltrated with 1x PBS (pH 7.4) and further concentrated. The Zₐᵥₑ of the resulting lipid nanoparticles was 115.5nm (Dv₍₅₀₎ = 92.1nm; Dv₍₉₀₎ = 162nm) and the average Zeta potential was 29.1mV. The lipid nanoparticles demonstrated an encapsulation efficiency of approximately 80%.

The lipid nanoparticles were then exposed to aqueous solutions comprising the reducing agent β-mercaptoethanol (β-ME) and the Zeta potential of the lipid nanoparticle assessed. Exposure of the lipid nanoparticles to 25µL (10X) of a β-ME solution for eighteen hours caused the Zeta potential of the lipid nanoparticle to be reduced to -3.50mV. Exposure of the lipid nanoparticles to 250µL (100X) of a β-ME solution for one hour caused the Zeta potential of the lipid nanoparticle to be reduced to 14.8mV. Exposure of the lipid nanoparticles to 250µL (100X) of a β-ME solution for two and one-half hours caused the Zeta potential of the lipid nanoparticle to be reduced to 2.85mV. Exposure of the lipid nanoparticles to 250µL (100X) of a β-ME solution for three hours caused the Zeta potential of the lipid nanoparticle to be reduced to -3.40mV. The foregoing supports the conclusion that by exposing charged lipid nanoparticles to a reducing agent, the charge (i.e., Zeta potential) of the lipid nanoparticle can be modulated, and in certain instances neutralized.

### Example 3

The present example further illustrates that HGT4002-based lipid nanoparticles may be reductively neutralized after having been loaded with mRNA polynucleotide constructs. The HGT4002-based lipid nanoparticles were formed via standard ethanol injection methods as described below. (Ponsa, et al., Int. J. Pharm. (1993) 95: 51-56.) Ethanolic stock solutions of the lipids were prepared ahead of time at a concentration of 50 mg/mL and stored at -20°C.

Codon-optimized firefly luciferase (CO-FFL) mRNA was synthesized by *in vitro* transcription from a plasmid DNA template, which was followed by the addition of a 5' cap structure (Cap1) (Fechter, P. et al., J. Gen. Virology (2005) 86: 1239-1249) and a 3' poly(A) tail of approximately 200 nucleotides in length as determined by gel electrophoresis. The 5' and 3' untranslated regions present in each mRNA product are represented as X and Y, respectively in SEQ ID NO: 1, as indicated below.
*Codon-Optimized Firefly Luciferase (FFL) mRNA:* **SEQ ID NO: 1** 
XAUGGAAGAUGCCAAAAACAUUAAGAAGGGCCCAGCGCCAUUCUACCCACUCGAAGACGGGA
CCGCCGGCGAGCAGCUGCACAAAGCCAUGAAGCGCUACGCCCUGGUGCCCGGCACCAUCGCC
UUUACCGACGCACAUAUCGAGGUGGACAUUACCUACGCCGAGUACUUCGAGAUGAGCGUUCG
GCUGGCAGAAGCUAUGAAGCGCUAUGGGCUGAAUACAAACCAUCGGAUCGUGGUGUGCAGCG
AGAAUAGCUUGCAGUUCUUCAUGCCCGUGUUGGGUGCCCUGUUCAUCGGUGUGGCUGUGGCC
CCAGCUAACGACAUCUACAACGAGCGCGAGCUGCUGAACAGCAUGGGCAUCAGCCAGCCCAC
CGUCGUAUUCGUGAGCAAGAAAGGGCUGCAAAAGAUCCUCAACGUGCAAAAGAAGCUACCGA
UCAUACAAAAGAUCAUCAUCAUGGAUAGCAAGACCGACUACCAGGGCUUCCAAAGCAUGUAC
ACCUUCGUGACUUCCCAUUUGCCACCCGGCUUCAACGAGUACGACUUCGUGCCCGAGAGCUU
CGACCGGGACAAAACCAUCGCCCUGAUCAUGAACAGUAGUGGCAGUACCGGAUUGCCCAAGG
GCGUAGCCCUACCGCACCGCACCGCUUGUGUCCGAUUCAGUCAUGCCCGCGACCCCAUCUUC
 
GGCAACCAGAUCAUCCCCGACACCGCUAUCCUCAGCGUGGUGCCAUUUCACCACGGCUUCGG
CAUGUUCACCACGCUGGGCUACUUGAUCUGCGGCUUUCGGGUCGUGCUCAUGUACCGCUUCG
AGGAGGAGCUAUUCUUGCGCAGCUUGCAAGACUAUAAGAUUCAAUCUGCCCUGCUGGUGCCC
ACACUAUUUAGCUUCUUCGCUAAGAGCACUCUCAUCGACAAGUACGACCUAAGCAACUUGCA
CGAGAUCGCCAGCGGCGGGGCGCCGCUCAGCAAGGAGGUAGGUGAGGCCGUGGCCAAACGCU
UCCACCUACCAGGCAUCCGCCAGGGCUACGGCCUGACAGAAACAACCAGCGCCAUUCUGAUC
ACCCCCGAAGGGGACGACAAGCCUGGCGCAGUAGGCAAGGUGGUGCCCUUCUUCGAGGCUAA
GGUGGUGGACUUGGACACCGGUAAGACACUGGGUGUGAACCAGCGCGGCGAGCUGUGCGUCC
GUGGCCCCAUGAUCAUGAGCGGCUACGUUAACAACCCCGAGGCUACAAACGCUCUCAUCGAC
AAGGACGGCUGGCUGCACAGCGGCGACAUCGCCUACUGGGACGAGGACGAGCACUUCUUCAU
CGUGGACCGGCUGAAGAGCCUGAUCAAAUACAAGGGCUACCAGGUAGCCCCAGCCGAACUGG
AGAGCAUCCUGCUGCAACACCCCAACAUCUUCGACGCCGGGGUCGCCGGCCUGCCCGACGAC
GAUGCCGGCGAGCUGCCCGCCGCAGUCGUCGUGCUGGAACACGGUAAAACCAUGACCGAGAA
GGAGAUCGUGGACUAUGUGGCCAGCCAGGUUACAACCGCCAAGAAGCUGCGCGGUGGUGUUG
UGUUCGUGGACGAGGUGCCUAAAGGACUGACCGGCAAGUUGGACGCCCGCAAGAUCCGCGAG
AUUCUCAUUAAGGCCAAGAAGGGCGGCAAGAUCGCCGUGUAY
**X** = GGGAUCCUACC **(SEQ ID NO: 2)**
**Y** = UUUGAAUU **(SEQ ID NO: 3)**

Next, 0.25mg of the FFL mRNA was denatured by heating it at 70°C for 5 minutes using a heat block. A 3mL volume of an aqueous buffered solution (10 mM citrate/150 mM NaCl, pH 4.5) was prepared and heated in an RNase-Free conical tube by placing the tube in a heated water bath, and into which was followed by the instant addition of the FFL mRNA into the heated aqueous buffered solution to achieve a final FFL mRNA concentration of 0.25mg/3mL.

All mRNA concentrations were determined via the Ribogreen assay (Invitrogen). Encapsulation of mRNA was calculated by performing the Ribogreen assay with and without the presence of 0.1% Triton-X 100. Particle sizes (dynamic light scattering (DLS)) and zeta potentials were determined using a Malvern Zetasizer instrument in 1x PBS and 1mM KCl solutions, respectively.

To prepare the lipid nanoparticles, aliquots of 50 mg/mL ethanolic solutions of HGT4002, DOPE, cholesterol and DMG-PEG2000 were mixed as indicated in Table 3 below, heated to ensure dissolution and diluted with ethanol. The resulting lipid solution is suitable for 0.25mg mRNA at N/P ratio of 4.

**Table 3**

| **Lipid Components** | **Molar Ratio** | **MW** | **µmol** | **Weight (mg)** | **Volume (µL)** |
|---|---|---|---|---|---|
| DMG-PEG2000 | 6 | 2509.19 | 1.04 | 2.6 | 52.2 |
| HGT4002 | 18 | 506 | 3.12 | 1.6 | 31.6 |
| CHOL | 20 | 386.65 | 3.47 | 1.3 | 26.8 |
| DOPE | 56 | 744.03 | 9.71 | 7.2 | 144.4 |
| *Total* | *100* | -- | *17.33* | *12.8* | *255.0* |

0.5mL of the ethanolic lipid solution was injected rapidly into the heated (70°C) aqueous mRNA/buffer solution, shaken and the resulting suspension quickly returned to the 70°C water bath. The resulting nanoparticle suspension was concentrated, re-suspended in a sodium borate buffer (pH 8.0), passed through MUSTANG Q membranes (Pall Life Sciences), was filtered and diafiltrated with 1x PBS (pH 7.4) and further concentrated. The average Zeta potential of the resulting lipid nanoparticles was 25.7mV.

A 0.1M stock solution comprising the reducing agent *tris* (2-carboxyethyl)phosphine (TCEP) was prepared by dissolving 28.7mg TCEP in 1mL of water. The lipid nanoparticles were then exposed to aqueous solutions comprising the TCEP and the Zeta potential of the lipid nanoparticle assessed. Exposure of the lipid nanoparticles to 19.5µL (5X) of a TCEP solution for five minutes caused the Zeta potential of the lipid nanoparticle to increase to 26.1mV. Exposure of the lipid nanoparticles to 19.5µL (5X) of a TCEP solution for two hours caused the Zeta potential of the lipid nanoparticle to be reduced to 14.5mV. Exposure of the lipid nanoparticles to 19.5µL (5X) of a TCEP solution for four hours caused the Zeta potential of the lipid nanoparticle to be reduced to 5.65mV. It is likely that the concentration of the TCEP solution and the duration of exposure contributed to the observed changes in the Zeta potential observed in a relatively short period of time.

The foregoing supports the conclusion that upon exposure to a reducing agent, the charge (i.e., Zeta potential) of HGT4002-based lipid nanoparticles can be modulated, and in certain instances neutralized.

### Example 4

The present example illustrates that HGT4002-based lipid nanoparticles may be reductively neutralized after having been loaded with mRNA polynucleotide constructs. The HGT4002-based lipid nanoparticles were formed via standard ethanol injection methods as described below. (Ponsa, et al., Int. J. Pharm. (1993) 95: 51-56.) Ethanolic stock solutions of the lipids were prepared ahead of time at a concentration of 50 mg/mL and stored at -20°C.

Codon-optimized firefly luciferase (CO-FFL) mRNA was synthesized by *in vitro* transcription from a plasmid DNA template encoding the gene, which was followed by the addition of a 5' cap structure (Cap1) (Fechter, P. et al., J. Gen. Virology (2005) 86: 1239-1249) and a 3' poly(A) tail of approximately 200 nucleotides in length as determined by gel electrophoresis. The 5' and 3' untranslated regions present in each mRNA product are represented as X and Y, respectively in SEQ ID NO: 1, as indicated below.
*Codon-Optimized Firefly Luciferase (FFL) mRNA:* **SEQ ID NO: 1** 
XAUGGAAGAUGCCAAAAACAUUAAGAAGGGCCCAGCGCCAUUCUACCCACUCGAAGACGGGA
CCGCCGGCGAGCAGCUGCACAAAGCCAUGAAGCGCUACGCCCUGGUGCCCGGCACCAUCGCC
UUUACCGACGCACAUAUCGAGGUGGACAUUACCUACGCCGAGUACUUCGAGAUGAGCGUUCG
GCUGGCAGAAGCUAUGAAGCGCUAUGGGCUGAAUACAAACCAUCGGAUCGUGGUGUGCAGCG
AGAAUAGCUUGCAGUUCUUCAUGCCCGUGUUGGGUGCCCUGUUCAUCGGUGUGGCUGUGGCC
CCAGCUAACGACAUCUACAACGAGCGCGAGCUGCUGAACAGCAUGGGCAUCAGCCAGCCCAC
CGUCGUAUUCGUGAGCAAGAAAGGGCUGCAAAAGAUCCUCAACGUGCAAAAGAAGCUACCGA
UCAUACAAAAGAUCAUCAUCAUGGAUAGCAAGACCGACUACCAGGGCUUCCAAAGCAUGUAC
ACCUUCGUGACUUCCCAUUUGCCACCCGGCUUCAACGAGUACGACUUCGUGCCCGAGAGCUU
CGACCGGGACAAAACCAUCGCCCUGAUCAUGAACAGUAGUGGCAGUACCGGAUUGCCCAAGG
GCGUAGCCCUACCGCACCGCACCGCUUGUGUCCGAUUCAGUCAUGCCCGCGACCCCAUCUUC
GGCAACCAGAUCAUCCCCGACACCGCUAUCCUCAGCGUGGUGCCAUUUCACCACGGCUUCGG
CAUGUUCACCACGCUGGGCUACUUGAUCUGCGGCUUUCGGGUCGUGCUCAUGUACCGCUUCG
AGGAGGAGCUAUUCUUGCGCAGCUUGCAAGACUAUAAGAUUCAAUCUGCCCUGCUGGUGCCC
ACACUAUUUAGCUUCUUCGCUAAGAGCACUCUCAUCGACAAGUACGACCUAAGCAACUUGCA
CGAGAUCGCCAGCGGCGGGGCGCCGCUCAGCAAGGAGGUAGGUGAGGCCGUGGCCAAACGCU
UCCACCUACCAGGCAUCCGCCAGGGCUACGGCCUGACAGAAACAACCAGCGCCAUUCUGAUC
ACCCCCGAAGGGGACGACAAGCCUGGCGCAGUAGGCAAGGUGGUGCCCUUCUUCGAGGCUAA
GGUGGUGGACUUGGACACCGGUAAGACACUGGGUGUGAACCAGCGCGGCGAGCUGUGCGUCC
GUGGCCCCAUGAUCAUGAGCGGCUACGUUAACAACCCCGAGGCUACAAACGCUCUCAUCGAC
AAGGACGGCUGGCUGCACAGCGGCGACAUCGCCUACUGGGACGAGGACGAGCACUUCUUCAU
CGUGGACCGGCUGAAGAGCCUGAUCAAAUACAAGGGCUACCAGGUAGCCCCAGCCGAACUGG
AGAGCAUCCUGCUGCAACACCCCAACAUCUUCGACGCCGGGGUCGCCGGCCUGCCCGACGAC
GAUGCCGGCGAGCUGCCCGCCGCAGUCGUCGUGCUGGAACACGGUAAAACCAUGACCGAGAA
GGAGAUCGUGGACUAUGUGGCCAGCCAGGUUACAACCGCCAAGAAGCUGCGCGGUGGUGUUG
UGUUCGUGGACGAGGUGCCUAAAGGACUGACCGGCAAGUUGGACGCCCGCAAGAUCCGCGAG
AUUCUCAUUAAGGCCAAGAAGGGCGGCAAGAUCGCCGUGUAY
**X** = GGGAUCCUACC **(SEQ ID NO: 2)**
**Y** = UUUGAAUU **(SEQ ID NO: 3)**

Next, 0.25mg of the FFL mRNA was denatured by heating it at 70°C for 5 minutes using a heat block. An 11mL volume of an aqueous buffered solution (10 mM citrate/150 mM NaCl, pH 4.5) was prepared and heated in a 50mL RNase-Free conical tube by placing the tube in a heated water bath, and into which was followed by the instant addition of the denatured CO-FFL mRNA into the heated aqueous buffered solution to achieve a final FFL mRNA concentration of 1mg/11mL.

All mRNA concentrations were determined via the Ribogreen assay (Invitrogen). Encapsulation of mRNA was calculated by performing the Ribogreen assay with and without the presence of 0.1% Triton-X 100. Particle sizes (dynamic light scattering (DLS)) and zeta potentials were determined using a Malvern Zetasizer instrument in 1x PBS and 1mM KCl solutions, respectively.

To prepare the lipid nanoparticles, aliquots of 50 mg/mL ethanolic solutions of HGT4002, DOPE, cholesterol and DMG-PEG2000 were mixed as indicated in Table 4 below, heated to ensure dissolution and diluted with ethanol. The resulting lipid solution is suitable for 0.25mg mRNA at N/P ratio of 4.

**Table 4**

| **Lipid Components** | **Molar Ratio** | **MW** | **µmol** | **Weight (mg)** | **Volume (µL)** |
|---|---|---|---|---|---|
| DMG-PEG2000 | 10 | 2509.19 | 2.50 | 6.3 | 125.5 |
| HGT4002 | 50 | 506 | 12.50 | 6.3 | 126.5 |
| CHOL | 20 | 386.65 | 5.0 | 1.9 | 38.7 |
| DOPE | 20 | 744.03 | 5.0 | 3.7 | 74.4 |
| *Total* | *100* | -- | *25.00* | *18.3* | *365.0* |

3.0mL of the ethanolic lipid solution was injected rapidly into the heated (70°C) aqueous mRNA/buffer solution, shaken and the resulting suspension quickly returned to the 70°C water bath. The resulting nanoparticle suspension was concentrated, re-suspended in a sodium borate buffer (pH 8.0), passed through MUSTANG Q membranes (Pall Life Sciences), was filtered and diafiltrated with 1x PBS (pH 7.4) and further concentrated. The Zₐᵥₑ of the resulting lipid nanoparticles was 58.40 nm (Dv₍₅₀₎ = 44.7nm; Dv₍₉₀₎ = 65.1nm) and the average Zeta potential was +7.8mV. Encapsulation of mRNA was calculated by performing the Ribogreen assay with and without the presence of 0.1% Triton-X 100 and determined to be 88.56%.

A 0.1M stock solution comprising the reducing agent *tris* (2-carboxyethyl)phosphine (TCEP) was prepared by dissolving 28.7mg TCEP in 1mL of water. The lipid nanoparticles were then exposed to aqueous solutions comprising the TCEP and the Zeta potential of the lipid nanoparticle assessed. Exposure of the lipid nanoparticles to 39µL (10X) of a TCEP solution for three hours caused the Zeta potential of the lipid nanoparticle to decrease to -17.1mV. Exposure of the lipid nanoparticles to 19.5µL (5X) of a TCEP solution for one hour caused the Zeta potential of the lipid nanoparticle to be reduced to -4.15mV. The foregoing supports the conclusion that upon exposure to a reducing agent, the charge (i.e., Zeta potential) of HGT4002-based lipid nanoparticles can be modulated, and in certain instances neutralized.

### Example 5

The present example illustrates that HGT4002-based lipid nanoparticles may be reductively neutralized after having been loaded with mRNA polynucleotide constructs. The HGT4002-based lipid nanoparticles were formed via standard ethanol injection methods as described below. (Ponsa, et al., Int. J. Pharm. (1993) 95: 51-56.) Ethanolic stock solutions of the lipids were prepared ahead of time at a concentration of 50 mg/mL and stored at -20°C.

Codon-optimized firefly luciferase (CO-FFL) mRNA was synthesized by *in vitro* transcription from a plasmid DNA template encoding the gene, which was followed by the addition of a 5' cap structure (Cap1) (Fechter, P. et al., J. Gen. Virology (2005) 86: 1239-1249) and a 3' poly(A) tail of approximately 200 nucleotides in length as determined by gel electrophoresis. The 5' and 3' untranslated regions present in each mRNA product are represented as X and Y, respectively in SEQ ID NO: 1, as listed below.
*Codon-Optimized Firefly Luciferase (FFL) mRNA:* **SEQ ID NO: 1** 
XAUGGAAGAUGCCAAAAACAUUAAGAAGGGCCCAGCGCCAUUCUACCCACUCGAAGACGGGA
CCGCCGGCGAGCAGCUGCACAAAGCCAUGAAGCGCUACGCCCUGGUGCCCGGCACCAUCGCC
UUUACCGACGCACAUAUCGAGGUGGACAUUACCUACGCCGAGUACUUCGAGAUGAGCGUUCG
 
GCUGGCAGAAGCUAUGAAGCGCUAUGGGCUGAAUACAAACCAUCGGAUCGUGGUGUGCAGCG
AGAAUAGCUUGCAGUUCUUCAUGCCCGUGUUGGGUGCCCUGUUCAUCGGUGUGGCUGUGGCC
CCAGCUAACGACAUCUACAACGAGCGCGAGCUGCUGAACAGCAUGGGCAUCAGCCAGCCCAC
CGUCGUAUUCGUGAGCAAGAAAGGGCUGCAAAAGAUCCUCAACGUGCAAAAGAAGCUACCGA
UCAUACAAAAGAUCAUCAUCAUGGAUAGCAAGACCGACUACCAGGGCUUCCAAAGCAUGUAC
ACCUUCGUGACUUCCCAUUUGCCACCCGGCUUCAACGAGUACGACUUCGUGCCCGAGAGCUU
CGACCGGGACAAAACCAUCGCCCUGAUCAUGAACAGUAGUGGCAGUACCGGAUUGCCCAAGG
GCGUAGCCCUACCGCACCGCACCGCUUGUGUCCGAUUCAGUCAUGCCCGCGACCCCAUCUUC
GGCAACCAGAUCAUCCCCGACACCGCUAUCCUCAGCGUGGUGCCAUUUCACCACGGCUUCGG
CAUGUUCACCACGCUGGGCUACUUGAUCUGCGGCUUUCGGGUCGUGCUCAUGUACCGCUUCG
AGGAGGAGCUAUUCUUGCGCAGCUUGCAAGACUAUAAGAUUCAAUCUGCCCUGCUGGUGCCC
ACACUAUUUAGCUUCUUCGCUAAGAGCACUCUCAUCGACAAGUACGACCUAAGCAACUUGCA
CGAGAUCGCCAGCGGCGGGGCGCCGCUCAGCAAGGAGGUAGGUGAGGCCGUGGCCAAACGCU
UCCACCUACCAGGCAUCCGCCAGGGCUACGGCCUGACAGAAACAACCAGCGCCAUUCUGAUC
ACCCCCGAAGGGGACGACAAGCCUGGCGCAGUAGGCAAGGUGGUGCCCUUCUUCGAGGCUAA
GGUGGUGGACUUGGACACCGGUAAGACACUGGGUGUGAACCAGCGCGGCGAGCUGUGCGUCC
GUGGCCCCAUGAUCAUGAGCGGCUACGUUAACAACCCCGAGGCUACAAACGCUCUCAUCGAC
AAGGACGGCUGGCUGCACAGCGGCGACAUCGCCUACUGGGACGAGGACGAGCACUUCUUCAU
CGUGGACCGGCUGAAGAGCCUGAUCAAAUACAAGGGCUACCAGGUAGCCCCAGCCGAACUGG
AGAGCAUCCUGCUGCAACACCCCAACAUCUUCGACGCCGGGGUCGCCGGCCUGCCCGACGAC
GAUGCCGGCGAGCUGCCCGCCGCAGUCGUCGUGCUGGAACACGGUAAAACCAUGACCGAGAA
GGAGAUCGUGGACUAUGUGGCCAGCCAGGUUACAACCGCCAAGAAGCUGCGCGGUGGUGUUG
UGUUCGUGGACGAGGUGCCUAAAGGACUGACCGGCAAGUUGGACGCCCGCAAGAUCCGCGAG
AUUCUCAUUAAGGCCAAGAAGGGCGGCAAGAUCGCCGUGUAY
**X** = GGGAUCCUACC **(SEQ ID NO: 2)**
**Y** = UUUGAAUU **(SEQ ID NO: 3)**

Next, 0.25mg of the FFL mRNA was denatured by heating it at 70°C for 5 minutes using a heat block. An 11mL volume of an aqueous buffered solution (10 mM citrate/150 mM NaCl, pH 4.5) was prepared and heated in a 50mL RNase-Free conical tube by placing the tube in a heated water bath, and into which was followed by the instant addition of the denatured CO-FFL mRNA into the heated aqueous buffered solution to achieve a final FFL mRNA concentration of 1mg/11mL.

All mRNA concentrations were determined via the Ribogreen assay (Invitrogen). Encapsulation of mRNA was calculated by performing the Ribogreen assay with and without the presence of 0.1% Triton-X 100. Particle sizes (dynamic light scattering (DLS)) and zeta potentials were determined using a Malvern Zetasizer instrument in 1x PBS and 1mM KCl solutions, respectively.

To prepare the lipid nanoparticles, aliquots of 50 mg/mL ethanolic solutions of HGT4002, DOPE, cholesterol and DMG-PEG2000 were mixed as indicated below in Table 5, heated to ensure dissolution and diluted with ethanol. The resulting lipid solution is suitable for 0.25mg mRNA at N/P ratio of 4.

**Table 5**

| **Lipid Components** | **Molar Ratio** | **MW** | **µmol** | **Weight (mg)** | **Volume (µL)** |
|---|---|---|---|---|---|
| DMG-PEG2000 | 10 | 2509.19 | 2.50 | 6.3 | 125.5 |
| HGT4002 | 50 | 506 | 12.50 | 6.3 | 126.5 |
| CHOL | 20 | 386.65 | 5.0 | 1.9 | 38.7 |
| DOPE | 20 | 744.03 | 5.0 | 3.7 | 74.4 |
| *Total Ethanol (additional)* | *100* | -- | *25.00* | *18.3* | *365.0* |
| | -- | -- | -- | -- | *2635.0* |
| *Final Volume* | | | | | *3000.0* |

3.0mL of the ethanolic lipid solution was injected rapidly into the heated (70°C) aqueous mRNA/buffer solution, shaken and the resulting suspension quickly returned to the 70°C water bath. The resulting nanoparticle suspension was concentrated, re-suspended in a sodium borate buffer (pH 8.0), passed through MUSTANG Q membranes (Pall Life Sciences), was filtered and diafiltrated with 1x PBS (pH 7.4) and further concentrated. The Zₐᵥₑ of the resulting lipid nanoparticles was 66.43nm (Dv₍₅₀₎ = 38.7nm; Dv₍₉₀₎ = 67.9nm) and the average Zeta potential was approximately 25.0mV. Encapsulation of mRNA was calculated by performing the Ribogreen assay with and without the presence of 0.1% Triton-X 100 and determined to be 70.79%.

A 0.1M stock solution comprising the reducing agent *tris (2-*carboxyethyl)phosphine (TCEP) was prepared by dissolving 28.7mg TCEP in 1mL of water. The lipid nanoparticles were then exposed to aqueous solutions comprising the TCEP and the Zeta potential of the lipid nanoparticle assessed. Exposure of the lipid nanoparticles to 19.5µL (5X) of a TCEP solution for one hour caused the Zeta potential of the lipid nanoparticle to be reduced to 14.9mV. The foregoing supports the conclusion that upon exposure to a reducing agent, the charge (i.e., Zeta potential) of HGT4002-based lipid nanoparticles can be modulated, and in certain instances neutralized.

## Claims

1. A method of encapsulating one or more therapeutic agents in a neutral lipid nanoparticle, such method comprising the steps of: (a) loading a lipid nanoparticle comprising at least one reducible cationic lipid with the one or more therapeutic agents; and (b) contacting the lipid nanoparticle with one or more reducing agents such that the lipid nanoparticle is neutralized;
wherein said reducible cationic lipid has the structure: 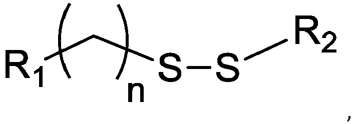
wherein R₁ is a releasable polar head-group and is selected from the group consisting of imidazole, guanidinium, imine, enamine, amino, an optionally-substituted alkyl amino and an optionally-substituted pyridyl;
wherein R₂ is selected from the group consisting of 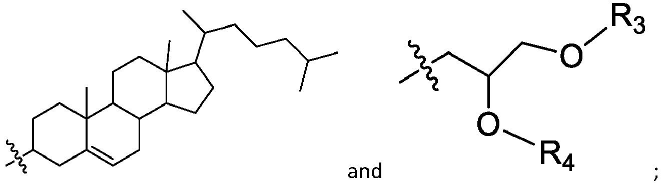
wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl;
wherein n is zero or any positive integer.

2. The method of claim 1, wherein the reducible cationic lipid has one of the following structures: 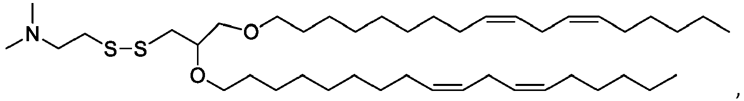 or 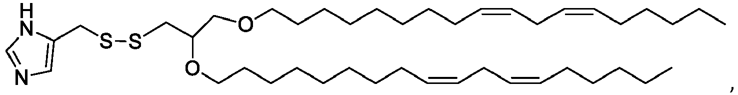 or

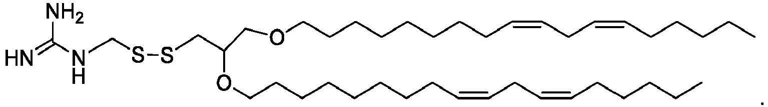

3. The method of claim 1, wherein the reducible cationic lipid has one of the following structures: 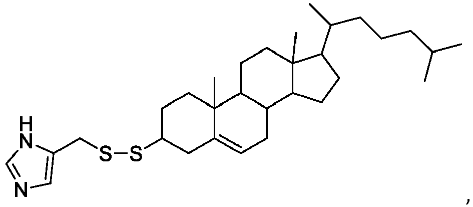 or

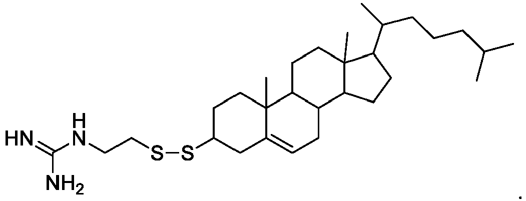

4. The method of claim 1, wherein the one or more reducing agents are selected from the group consisting of *tris* (2-carboxyethyl)phosphine (TCEP), β-mercaptoethanol (β-ME), dithiothreitol (DTT), glutathione, dithioerythritol and combinations thereof.

5. The method of claim 1, wherein the lipid nanoparticle is contacted with the reducing agent for at least five minutes, for at least 1 hour, for at least 3 hours or for at least 24 hours.

6. The method of claim 1, wherein the zeta potential of the lipid nanoparticle is at least 25mV prior to contacting the lipid nanoparticle with the reducing agent and less than 5mV, -25.0mV to 5.0mV, or -10.0 to 5.0mV after contacting the lipid nanoparticle with the reducing agent.

7. The method of claim 1, wherein the therapeutic agent comprises one or more polynucleotides.

8. The method of claim 7, wherein the polynucleotides have phosphorothioate internucleotide linkages at each internucleotide linkage.

9. The method of claim 7, wherein
(a) the one or more polynucleotides comprise mRNA, optionally wherein:
(i) the mRNA comprises one or more nucleotide modifications selected from the group consisting of: Locked Nucleic Acid (LNA); 2'-O-alkyl-RNA units, 2'-OMe-RNA units, 2'-amino-DNA units, and 2'-fluoro-DNA units, or
(ii) the mRNA comprises at least one modified nucleotide independently selected from the group consisting of 5-methylcytosine, isocytosine, pseudoisocytosine, 5-bromouracil, 5-propynyluracil, 6-aminopurine, 2-aminopurine, inosine, pseudouridine, 2-thiouridine, diaminopurine and 2-chloro-6-aminopurine cytosine, or
(b) the one or more polynucleotides are selected from the group consisting of an antisense oligonucleotide, siRNA, miRNA, snRNA, snoRNA and combinations thereof, or
(c) the one or more polynucleotide comprises one or more LNA, or
(d) the one or more polynucleotides comprise DNA.

10. The method of claim 7, wherein the one or more polynucleotides comprise RNA, optionally wherein:
(i) the RNA is selected from the group consisting of mRNA, siRNA, snoRNA, microRNA, and combinations thereof, or
(ii) the RNA encodes a protein or enzyme,
optionally wherein the protein or enzyme is selected from the group consisting of human growth hormone, erythropoietin, α1-antitrypsin, acid alpha glucosidase, arylsulfatase A, carboxypeptidase N, alpha galactosidase A, alpha-L-iduronidase, iduronate-2-sulfatase, iduronate sulfatase, N-acetylglucosamine-1-phosphate transferase, N-acetylglucosaminidase, alpha-glucosaminide acetyltransferase, N-acetylglucosamine 6-sulfatase, N-acetylgalactosamine-4-sulfatase, beta-glucosidase, galactose-6-sulfate sulfatase, beta-galactosidase, beta-glucuronidase, glucocerebrosidase, heparan sulfamidase, heparin-N-sulfatase, lysosomal acid lipase, hyaluronidase, galactocerebrosidase, ornithine transcarbamylase (OTC), carbamoyl-phosphate synthetase 1 (CPS1), argininosuccinate synthetase (ASS1), argininosuccinate lyase (ASL), arginase 1 (ARG1), cystic fibrosis transmembrane conductance regulator (CFTR), survival motor neuron (SMN), Factor IX, Factor VIII and low density lipoprotein receptors (LDLR).

11. The method of any one of claims 1-10, wherein the neutralized lipid nanoparticle comprises between 0.025µg/mL to 250µg/mL, for example at least 0.1µg/mL or at least 05µg/mL of encapsulated polynucleotide.

12. A neutral lipid nanoparticle having one or more encapsulated therapeutic agents prepared in accordance with the method of any one of claims 1-11.

13. A neutral lipid nanoparticle having one or more encapsulated therapeutic agents of claim 12 for use in therapy.

## Patentansprüche

1. Verfahren zur Verkapselung eines oder mehrerer therapeutischer Mittel in einem neutralen Lipidnanopartikel, wobei ein derartiges Verfahren die folgenden Schritte umfasst: (a) Beladen eines Lipidnanopartikels, der mindestens ein reduzierbares kationisches Lipid umfasst, mit dem einen oder den mehreren therapeutischen Mitteln; und b) Inkontaktbringen des Lipidnanopartikels mit einem oder mehreren Reduktionsmitteln, derart, dass der Lipidnanopartikel neutralisiert wird;
wobei das reduzierbare kationische Lipid die folgende Struktur hat: 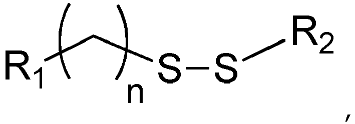
wobei R₁ eine abspaltbare polare Kopfgruppe ist und aus der Gruppe ausgewählt ist, die aus Imidazol, Guanidinium, Imin, Enamin, Amino, einem möglicherweise substituierten Alkylamino und einem möglicherweise substituierten Pyridyl besteht;
wobei R₂ aus der Gruppe ausgewählt ist, die aus Folgendem besteht 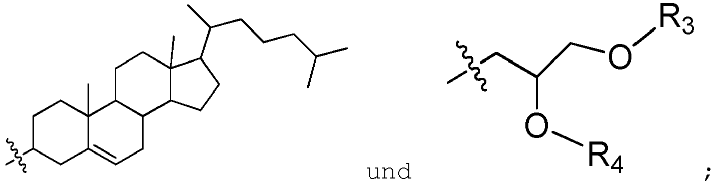
wobei R₃ und R₄ jeweils auf unabhängige Weise aus der Gruppe ausgewählt sind, die aus einem möglicherweise substituierten, wahlweise gesättigten oder ungesättigten C₆-C₂₀-Alkyl und einem möglicherweise substituierten, wahlweise gesättigten oder ungesättigten C₆-C₂₀-Acyl besteht;
wobei n gleich null ist oder für eine positive ganze Zahl steht.

2. Verfahren nach Anspruch 1, wobei das reduzierbare kationische Lipid eine der folgenden Strukturen hat: 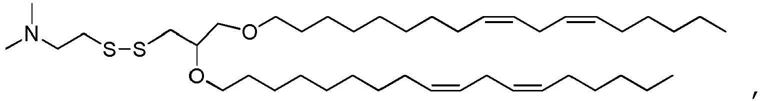 oder 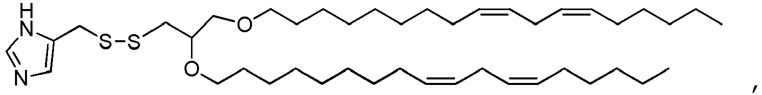 oder

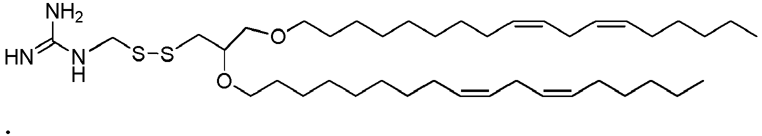

3. Verfahren nach Anspruch 1, wobei das reduzierbare kationische Lipid eine der folgenden Strukturen hat: 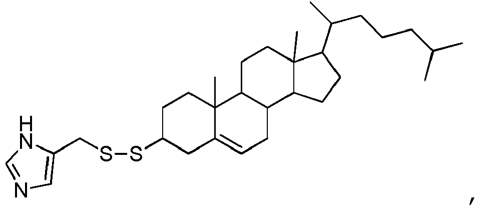 oder

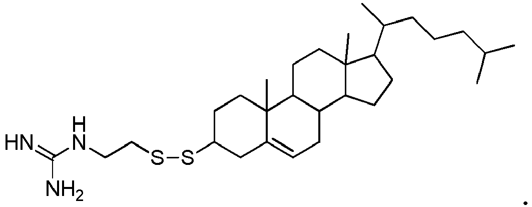

4. Verfahren nach Anspruch 1, wobei das eine oder die mehreren Reduktionsmittel aus der Gruppe ausgewählt sind, die aus *tris*-(2-Carboxyethyl)phosphin (TCEP), β-Mercaptoethanol (β-ME), Dithiothreit (DTT), Glutathion, Dithioerythrit und deren Kombinationen besteht.

5. Verfahren nach Anspruch 1, wobei der Lipidnanopartikel mindestens fünf Minuten lang, mindestens 1 Stunde lang, mindestens 3 Stunden lang oder mindestens 24 Stunden lang mit dem Reduktionsmittel in Kontakt gebracht wird.

6. Verfahren nach Anspruch 1, wobei das zeta-Potenzial des Lipidnanopartikel vor dem Inkontaktbringen des Lipidnanopartikels mit dem Reduktionsmittel mindestens 25 mV beträgt und nach dem Inkontaktbringen des Lipidnanopartikels mit dem Reduktionsmittel weniger als 5 mV, -25,0 mV bis 5,0 mV oder -10,0 bis 5,0 mV beträgt.

7. Verfahren nach Anspruch 1, wobei das therapeutische Mittel ein oder mehrere Polynukleotide umfasst.

8. Verfahren nach Anspruch 7, wobei die Polynukleotide, an jeder Internukleotidverknüpfung, Internukleotidverknüpfungen vom Typ Thiophosphat aufweisen.

9. Verfahren nach Anspruch 7, wobei
(a) das eine oder die mehreren Polynukleotide mRNA umfassen, wobei möglicherweise:
(i) die mRNA eine oder mehrere Nukleotidmodifizierungen umfasst, welche aus der Gruppe ausgewählt sind, die aus den folgenden besteht: verbrückte Nukleinsäure (LNA); 2'-O-Alkyl-RNA-Einheiten, 2'-OMe-RNA-Einheiten, 2'-Amino-DNA-Einheiten und 2'-Fluor-DNA-Einheiten, oder
(ii) die mRNA mindestens eines der modifizierten Nukleotide umfasst, welche auf unabhängige Weise aus der Gruppe ausgewählt sind, welche aus 5-Methylcytosin, Isocytosin, Pseudoisocytosin, 5-Bromuracil, 5-Propinyluracil, 6-Aminopurin, 2-Aminopurin, Inosin, Pseudouridin, 2-Thiouridin, Diaminopurin und 2-Chlor-6-aminopurin-Cytosin besteht, oder
(b) das eine oder die mehreren Polynukleotide aus der Gruppe ausgewählt sind, welche aus einem antisense-Oligonukleotid, siRNA, miRNA, snRNA, snoRNA und deren Kombinationen besteht, oder
(c) das Polynukleotid, von welchem eines oder mehrere vorliegen, eine oder mehrere LNA umfasst, oder
(d) das eine oder die mehreren Polynukleotide DNA umfassen.

10. Verfahren nach Anspruch 7, wobei das eine oder die mehreren Polynukleotide RNA umfassen, wobei möglicherweise:
(i) die RNA aus der Gruppe ausgewählt ist, die aus mRNA, siRNA, snoRNA, MikroRNA und deren Kombinationen besteht, oder
(ii) die RNA für ein Protein oder Enzym codiert, wobei das Protein oder Enzym möglicherweise aus der Gruppe ausgewählt ist, die aus einem Wachstumshormon des Menschen, Erythropoetin, α1-Antitrypsin, saurer alpha-Glucosidase, Arylsulfatase A, Carboxypeptidase N, alpha-Galactosidase A, alpha-L-Iduronidase, Iduronat-2-Sulfatase, Iduronat-Sulfatase, N-Acetylglucosamine-1-phosphat-Transferase, N-Acetylglucosaminidase, alpha-Glucosaminid-Acetyltransferase, N-Acetylglucosamin-6-Sulfatase, N-Acetylgalactosamin-4-Sulfatase, beta-Glucosidase, Galactose-6-sulfat-Sulfatase, beta-Galactosidase, beta-Glucuronidase, Glucocerebrosidase, Heparan-Sulfamidase, Heparin-N-Sulfatase, lysosomaler saurer Lipase, Hyaluronidase, Galactocerebrosidase, Ornithin-Transcarbamylase (OTC), Carbamoyl-Phosphat-Synthetase 1 (CPS1), Argininosuccinat-Synthetase (ASS1), Argininosuccinat-Lyase (ASL), Arginase 1 (ARG1), dem Cystic Fibrosis Transmembrane Conductance Regulator (CFTR), dem Survival Motor Neuron (SMN), dem Faktor IX, dem Faktor VIII sowie Lipoproteinrezeptoren niedriger Dichte (LDLR) besteht.

11. Verfahren nach einem beliebigen der Ansprüche 1 bis 10, wobei der neutralisierte Lipidnanopartikel zwischen 0,025 µg/mL und 250 µg/mL, beispielsweise mindestens 0,1 µg/mL oder mindestens 0,5 µg/mL, an verkapseltem Polynukleotid umfasst.

12. Neutraler Lipidnanopartikel, der ein oder mehrere verkapselte therapeutische Mittel aufweist, wobei er gemäß dem Verfahren nach einem beliebigen der Ansprüche 1 bis 11 hergestellt wurde.

13. Neutraler Lipidnanopartikel, der ein oder mehrere verkapselte therapeutische Mittel aufweist, nach Anspruch 12 zur therapeutischen Verwendung.

## Revendications

1. Procédé d'encapsulation d'un ou plusieurs agents thérapeutiques dans une nanoparticule lipidique neutre, un tel procédé comprenant les étapes de : (a) chargement d'une nanoparticule lipidique comprenant au moins un lipide cationique réductible avec l'agent ou les agents thérapeutiques ; et (b) mise en contact de la nanoparticule lipidique avec un ou plusieurs agents de réduction de sorte que la nanoparticule lipidique soit neutralisée ;
ledit lipide cationique réductible possédant la structure : 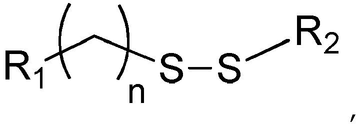
R₁ étant un groupe de tête polaire libérable et étant choisi dans le groupe constitué par imidazole, guanidinium, imine, énamine, amino, un alkylamino éventuellement substitué et un pyridinyle éventuellement substitué ;
R₂ étant choisi dans le groupe constitué par 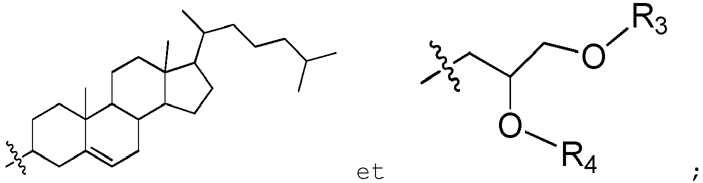
R₃ et R₄ étant chacun indépendamment choisis dans le groupe constitué par C₆₋₂₀-alkyle éventuellement substitué, saturé de manière variable ou insaturé et un C₆₋₂₀-acyle éventuellement substitué, saturé de manière variable ou insaturé ;
n étant zéro ou un entier positif.

2. Procédé selon la revendication 1, le lipide cationique réductible possédant l'une des structures suivantes : 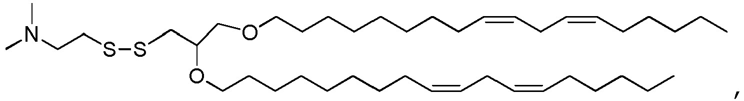 ou 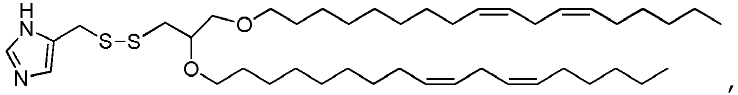 ou

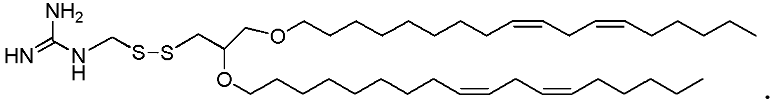

3. Procédé selon la revendication 1, le lipide cationique réductible possédant l'une des structures suivantes : 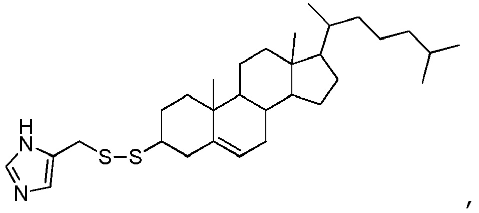 ou

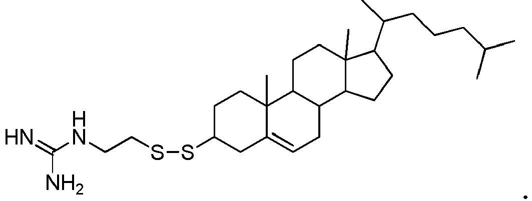

4. Procédé selon la revendication 1, l'agent ou les agents de réduction étant choisis dans le groupe constitué par la *tris*(2-carboxyéthyl)phosphine (TCEP), le β-mercaptoéthanol (β-ME), le dithiothréitol (DTT), le glutathion, le dithioérythritol et des combinaisons correspondantes.

5. Procédé selon la revendication 1, la nanoparticule lipidique étant mise en contact avec l'agent de réduction pendant au moins cinq minutes, pendant au moins 1 heure, pendant au moins 3 heures ou pendant au moins 24 heures.

6. Procédé selon la revendication 1, le potentiel zêta de la nanoparticule lipidique étant d'au moins 25 mV avant la mise en contact de la nanoparticule lipidique avec l'agent de réduction et moins de 5 mV, -25,0 mV à 5,0 mV, ou -10,0 à 5,0 mV après la mise en contact de la nanoparticule lipidique avec l'agent de réduction.

7. Procédé selon la revendication 1, l'agent thérapeutique comprenant un ou plusieurs polynucléotides.

8. Procédé selon la revendication 7, les polynucléotides possédant des liaisons internucléotidiques de type phosphorothioate au niveau de chaque liaison internucléotidique.

9. Procédé selon la revendication 7,
(a) le ou les polynucléotides comprenant un ARNm, éventuellement :
(i) l'ARNm comprenant une ou plusieurs modifications de nucléotide choisies dans le groupe constitué par : un acide nucléique bloqué (LNA) ; des motifs 2'-O-alkyl-ARN, des motifs 2'-OMe-ARN, des motifs 2'-amino-ADN, et des motifs 2'-fluoro-ADN, ou
(ii) l'ARNm comprenant au moins un nucléotide modifié indépendamment choisi dans le groupe constitué par la 5-méthylcytosine, l'isocytosine, la pseudoisocytosine, le 5-bromouracile, le 5-propynyluracile, la 6-aminopurine, la 2-aminopurine, l'inosine, la pseudouridine, la 2-thiouridine, la diaminopurine et la 2-chloro-6-aminopurine cytosine, ou
(b) le ou les polynucléotides étant choisis dans le groupe constitué par un oligonucléotide antisens, un ARNsi, un ARNmi, un ARNsn, un ARNsno et des combinaisons correspondantes, ou
(c) le ou les polynucléotides comprenant un ou plusieurs LNA, ou
(d) le ou les polynucléotides comprenant un ADN.

10. Procédé selon la revendication 7, le ou les polynucléotides comprenant un ARN, éventuellement :
(i) l'ARN étant choisi dans le groupe constitué par un ARNm, un ARNsi, un ARNsno, un ARNmicro, et des combinaisons correspondantes, ou
(ii) l'ARN codant pour une protéine ou une enzyme, éventuellement la protéine ou l'enzyme étant choisie dans le groupe constitué par une hormone de croissance humaine, l'érythropoïétine, la α1-antitrypsine, une alpha-glucosidase acide, l'arylsulfatase A, la carboxypeptidase N, l'alpha-galactosidase A, l'alpha-L-iduronidase, l'iduronate-2-sulfatase, l'iduronate sulfatase, la N-acétylglucosamine-1-phosphate transférase, la N-acétylglucosaminidase, l'alpha-glucosaminide acétyltransférase, la N-acétylglucosamine 6-sulfatase, la N-acétylgalactosamine-4-sulfatase, la bêta-glucosidase, la galactose-6-sulfate sulfatase, la bêta-galactosidase, la bêta-glucuronidase, la glucocérébrosidase, l'héparane sulfamidase, l'héparine-N-sulfatase, la lipase acide lysosomale, l'hyaluronidase, la galactocérébrosidase, l'ornithine transcarbamylase (OTC), la carbamoyl-phosphate synthétase 1 (CPS1), l'argininosuccinate synthétase (ASS1), l'argininosuccinate lyase (ASL), l'arginase 1 (ARG1), le régulateur de la conductance transmembranaire de la fibrose kystique (CFTR), le neurone moteur de survie (SMN), le Facteur IX, le facteur VIII et des récepteurs de lipoprotéine de basse densité (LDLR).

11. Procédé selon l'une quelconque des revendications 1 à 10, la nanoparticule lipidique neutralisée comprenant entre 0,025 µg/mL et 250 µg/mL, par exemple au moins 0,1 µg/mL ou au moins 0,5 µg/mL de polynucléotide encapsulé.

12. Nanoparticule lipidique neutre possédant un ou plusieurs agents thérapeutiques encapsulés préparée conformément au procédé selon l'une quelconque des revendications 1 à 11.

13. Nanoparticule lipidique neutre possédant un ou plusieurs agents thérapeutiques encapsulés selon la revendication 12 pour une utilisation en thérapie.


### SEQUENCE LISTING

<110> Shire Human Genetic Therapies Inc.
   DeRosa, Frank
   Guild, Braydon C.
   Heartlein, Michael
<120> Lipid-derived Neutral Nanoparticles
<130> 2006685-0287
<150> 61/617,478
   <151> 2012-03-29
<160> 3
<170> PatentIn version 3.5
<210> 1
   <211> 1671
   <212> RNA
   <213> Photinus pyralis
<400> 1 
gggauccuac cauggaagau gccaaaaaca uuaagaaggg cccagcgcca uucuacccac 60

ucgaagacgg gaccgccggc gagcagcugc acaaagccau gaagcgcuac gcccuggugc 120

ccggcaccau cgccuuuacc gacgcacaua ucgaggugga cauuaccuac gccgaguacu 180

ucgagaugag cguucggcug gcagaagcua ugaagcgcua ugggcugaau acaaaccauc 240

ggaucguggu gugcagcgag aauagcuugc aguucuucau gcccguguug ggugcccugu 300

ucaucggugu ggcuguggcc ccagcuaacg acaucuacaa cgagcgcgag cugcugaaca 360

gcaugggcau cagccagccc accgucguau ucgugagcaa gaaagggcug caaaagaucc 420

ucaacgugca aaagaagcua ccgaucauac aaaagaucau caucauggau agcaagaccg 480

acuaccaggg cuuccaaagc auguacaccu ucgugacuuc ccauuugcca cccggcuuca 540

acgaguacga cuucgugccc gagagcuucg accgggacaa aaccaucgcc cugaucauga 600

acaguagugg caguaccgga uugcccaagg gcguagcccu accgcaccgc accgcuugug 660

uccgauucag ucaugcccgc gaccccaucu ucggcaacca gaucaucccc gacaccgcua 720

uccucagcgu ggugccauuu caccacggcu ucggcauguu caccacgcug ggcuacuuga 780

ucugcggcuu ucgggucgug cucauguacc gcuucgagga ggagcuauuc uugcgcagcu 840

ugcaagacua uaagauucaa ucugcccugc uggugcccac acuauuuagc uucuucgcua 900

agagcacucu caucgacaag uacgaccuaa gcaacuugca cgagaucgcc agcggcgggg 960

cgccgcucag caaggaggua ggugaggccg uggccaaacg cuuccaccua ccaggcaucc 1020

gccagggcua cggccugaca gaaacaacca gcgccauucu gaucaccccc gaaggggacg 1080

acaagccugg cgcaguaggc aagguggugc ccuucuucga ggcuaaggug guggacuugg 1140

acaccgguaa gacacugggu gugaaccagc gcggcgagcu gugcguccgu ggccccauga 1200
 
ucaugagcgg cuacguuaac aaccccgagg cuacaaacgc ucucaucgac aaggacggcu 1260

ggcugcacag cggcgacauc gccuacuggg acgaggacga gcacuucuuc aucguggacc 1320

ggcugaagag ccugaucaaa uacaagggcu accagguagc cccagccgaa cuggagagca 1380

uccugcugca acaccccaac aucuucgacg ccggggucgc cggccugccc gacgacgaug 1440

ccggcgagcu gcccgccgca gucgucgugc uggaacacgg uaaaaccaug accgagaagg 1500

agaucgugga cuauguggcc agccagguua caaccgccaa gaagcugcgc ggugguguug 1560

uguucgugga cgaggugccu aaaggacuga ccggcaaguu ggacgcccgc aagauccgcg 1620

agauucucau uaaggccaag aagggcggca agaucgccgu guauuugaau u 1671
<210> 2
   <211> 11
   <212> RNA
   <213> Homo sapiens
<400> 2
   gggauccuac c 11
<210> 3
   <211> 8
   <212> RNA
   <213> Homo sapiens
<400> 3
   uuugaauu 8